# EUROPÄISCHE PATENTANMELDUNG

(11) **EP 3 407 058 A1**
(43) Veröffentlichungstag der Anmeldung: **28.11.2018**
(21) Anmeldenummer: 18173141.5
(22) Anmeldetag: 18.05.2018
(51) Int. Cl.: G01N 27/28, A61L 2/28, G01D 11/24

(54) **INLINE-SENSORANORDNUNG, VERFAHREN ZUR HERSTELLUNG UND INBETRIEBNAHME DESSELBEN**

(30) Priorität: 22.05.2017 DE 102017111141
(71) Anmelder: Endress + Hauser Conducta GmbH+Co. KG, 70839 Gerlingen (DE)
(72) Erfinder: Eubisch, Angela, 09120 Chemnitz (DE); Hanko, Michael, 01324 Dresden (DE)
(74) Vertreter: Andres, Angelika Maria

(57) **Zusammenfassung**

Die Erfindung betrifft eine Inline-Sensoranordnung zur Erfassung von Messwerten einer einen Analytgehalt eines Messmediums repräsentierenden Messgröße, welche umfasst: einen Sensor, welcher dazu ausgestaltet ist, ein mit der Messgröße korreliertes Messsignal zu erzeugen und auszugeben, wobei der Sensor mindestens ein zum Kontakt mit dem Messmedium vorgesehenes steriles Sensorelement aufweist; und ein das mindestens eine Sensorelement umgebendes Gehäuse, das das Sensorelement in einer gegenüber einer Umgebung des Gehäuses dicht verschlossenen Kammer einschließt, und wobei die Kammer in ihrem Innern ein Gasvolumen enthält, das derart ausgestaltet ist, dass ein Einfluss von Schadsubstanzen, insbesondere von reaktiven Stickstoff- und/oder Sauerstoff-Spezies, auf das mindestens eine Sensorelement weitgehend verhindert wird.

Die Erfindung betrifft weiter ein Verfahren zur Herstellung desselben und dessen Inbetriebnahme.

## Beschreibung

Die Erfindung betrifft eine Inline-Sensoranordnung zur Erfassung von Messwerten einer einen Analytgehalt eines Messmediums repräsentierenden Messgröße. Die Erfindung betrifft weiter ein Verfahren zur Herstellung desselben und zu dessen Inbetriebnahme.

Zur Bestimmung der Zusammensetzung von Messmedien, insbesondere Flüssigkeiten, wie z.B. reinen Flüssigkeiten, Flüssigkeitsgemischen, Emulsionen oder Suspensionen, werden in der Prozessmesstechnik und in der Analyse-Messtechnik vielfältige Analyse-Messgeräte eingesetzt. Ein Analyse-Messgerät umfasst im Allgemeinen einen Sensor, welcher dazu ausgestaltet ist, ein von mindestens einer Analyse-Messgröße des Messmediums abhängiges elektrisches Messsignal zu erzeugen, sowie eine Auswerteelektronik, die aus dem Messsignal einen den aktuellen Wert der mindestens einen Analyse-Messgröße im Messmedium repräsentierenden Messwert ermittelt. Die Analyse-Messgröße kann beispielsweise eine Konzentration oder Aktivität eines Analyten oder eine von einer Konzentration oder Aktivität mindestens eines Analyten in dem Messmedium abhängiger Parameter sein. Unter einem Analyten versteht man eine oder mehrere in dem Messmedium enthaltene, insbesondere gelöste, Substanz bzw. Substanzen, deren Konzentration in dem Messmedium mittels des Sensors ermittelt bzw. überwacht werden soll. Die Auswerteelektronik kann mindestens teilweise in einen direkt an der Messstelle angeordneten Messumformer integriert sein, der über ein Gehäuse mit Anzeige- und Eingabemitteln verfügt. Mindestens ein Teil der Auswerteelektronik kann auch mit dem Sensor zusammen in einem gemeinsamen Gehäuse angeordnet sein.

Solche Analyse-Messgeräte werden in vielfältigen Bereichen eingesetzt, z.B. zur Überwachung und Steuerung von Prozessen in der pharmazeutischen, chemischen, biotechnologischen oder biochemischen Produktion, aber auch in Prozessen der Wasseraufbereitung oder Abwasserreinigung, sowie in der Umweltanalytik. Soweit ein Analyse-Messgerät in einem Prozess eingesetzt wird, ist das Messmedium in der Regel in einem Prozessbehälter enthalten. Ein solcher Prozessbehälter kann z.B. eine Rohrleitung einer Prozessanlage oder ein Reaktionsgefäß, beispielsweise ein Fermenter, sein.

Sensoren, die in die Wandung eines Prozessbehälters zur Überwachung einer Messgröße eines in dem Prozessbehälter enthaltenen Messmediums integriert sind, bezeichnet man als Inline-Sensoren. Ein Inline-Sensor erfasst die Messgröße direkt in dem zu überwachenden Messmedium. Bei Inline-Sensoren ist daher zur Bestimmung einer Analyse-Messgröße keine Entnahme und Vorbehandlung einer Probe aus dem Prozess erforderlich. Zur Integration eines Sensors in die Prozesswandung sind vielfältige Adapter und Armaturen, insbesondere Tauch- oder Wechselarmaturen, bekannt. Eine Anordnung, die einen in der Wandung eines Prozessbehälters integrierten Inline-Sensor und gegebenenfalls eine mit dem Inline-Sensor verbundene, aber von diesem abgesetzte, Auswerteelektronik umfasst, wird als Inline-Sensoranordnung bezeichnet. Der Inline-Sensor kann in der Wandung mittels eines geeigneten Adapters befestigt sein.

Bei Prozessen, die unter sterilen bzw. aseptischen Bedingungen durchgeführt werden müssen, wie sie beispielsweise in der Biotechnologie, Pharmazie oder Lebensmitteltechnologie auftreten, werden vor Beginn des Prozesses oder zwischen einzelnen Prozessschritten alle mit den Prozessmedien in Berührung tretenden Teile der Prozessanlage, insbesondere alle Prozessbehälter und auch die darin integrierten Sensoren, in der Regel sterilisiert, beispielsweise thermisch durch Hitze. Die Hitzesterilisierung kann durch trockene Hitze (üblicherweise mit Heißluft zwischen 160 °C und 180 °C als Sterilisationsmedium) oder durch Heißdampf als Sterilisationsmedium unter Druckerhöhung erfolgen, beispielsweise durch Autoklavieren in einem Druckbehälter, einem so genannten Autoklaven. Gängig sind beispielsweise Heißdampf-Sterilisationsverfahren, bei denen Temperaturen von mindestens 120 °C oder mehr auftreten können. Wird die Hitzesterilisierung in einem Autoklaven durchgeführt, werden die prozessberührenden Teile der Prozessanlage, ggfs. bereits miteinander verbunden, in den Autoklaven eingebracht und dort sterilisiert. Die sterilisierten Teile werden anschließend wieder aus dem Autoklaven entnommen und in Betrieb genommen. Alternativ kann eine Prozessanlage mittels eines sog. SIP-Verfahrens (SIP ist die Abkürzung für den englischen Fachbegriff "*sterilization in place*") sterilisiert werden, bei denen der zu sterilisierende Prozessbehälter einschließlich der darin integrierten Inline-Sensoranordnungen mit Heißdampf sterilisiert wird, der über eine vorgegebene Zeitspanne in den Prozessbehälter eingeleitet wird. Inline-Sensoranordnungen müssen daher den dabei auftretenden Bedingungen, wie hohen Temperaturen und erhöhten Drücken, ohne Funktionalitätsverlust standhalten können.

In der Bioprozessmesstechnik, beispielsweise zur Überwachung, Steuerung und/oder Regelung von biotechnologischen Prozessen, werden auch Sensoren eingesetzt, die biologische Erkennungselemente aufweisen, z.B. solche, die, ggfs. als Rezeptoren, den Analyten selektiv und spezifisch binden. Biologische Erkennungselemente können Proteine wie Enzyme oder Antikörper, DNA-/RNA-Fragmente, Zellorganellen oder ganze Zellen und Mikroorganismen sein. Solche Sensoren werden als Biosensoren bezeichnet. Nach einem typischen Heißdampf-Sterilisationsprozess liegen die Rezeptoren bzw. biologische Erkennungselemente solcher Biosensoren in der Regel mit stark verminderter Aktivität vor, zumeist irreversibel denaturiert, d.h. in ihrer nativen 3-D-Stuktur (Konformation) zerstört vor. Derartige Biosensoren können daher grundsätzlich nicht ohne weiteres als Inline-Sensoren in die Wandung eines Prozessbehälters eingesetzt und mit diesem mittels eines gängigen SIP-Verfahrens sterilisiert werden.

Viele Sensoren mit biologischen Erkennungselementen, z.B. solche, die aus mesophilen Organismen stammen, welche im Temperaturbereich von ca. 20-45 °C leben, dürfen keinen erhöhten Temperaturen unter SIP-Bedingungen, beispielsweise oberhalb von 80°C ausgesetzt werden, um ihre Funktionalität nicht einzubüßen.

In der Literatur sind sterilisierbare Biosensoren basierend auf amperometrischen Enzymsensoren beschrieben. In M. Phelps, Development of a regenerable glucose biosensor probe for bioprocess monitoring, Master Thesis, University of British Columbia, 1993, ist ein Überblick über Literatur zu solchen Sensoren angegeben. Darin beschriebene Strategien zur Gewährleistung einer Sterilisierbarkeit solcher Biosensoren unter Erhalt ihrer Funktionalität umfassen das Einbringen der auf einem, beispielsweise eine Arbeitselektrode umfassenden, Träger angeordneten temperatursensiblen Rezeptoren erst nach dem Sterilisationsprozess in einen Reaktionsraum innerhalb eines Sensorgehäuses, welcher durch eine für den jeweiligen Analyten permeable Membran gegenüber dem Prozessbehälter verschlossen ist. Die Membran stellt in diesem Fall die Sterilbarriere dar. Dabei können die Rezeptoren immobilisiert auf der nachträglich eingebrachten Arbeitselektrode oder in einer in dem Reaktionsraum aufgenommenen Lösung vorliegen. Beim Einbringen der Rezeptoren darf die Sterilbarriere nicht zerstört werden, was die Handhabung derartiger Inline-Sensoranordnungen schwierig macht.

Nachteilig an diesen aus der Literatur bekannten Inline-Sensoranordnungen ist neben der schwierigen Handhabung auch, dass eine schwankende Messperformance der Biosensoren zu beobachten ist. Ein Grund dafür ist, dass die Menge der nachträglich eingebrachten Rezeptoren schlecht reproduzierbar ist. Die bisher bekannten Inline-Sensoranordnungen, die Biosensoren umfassen, sind nicht praxistauglich, insbesondere nicht im Hinblick auf Anwendungen zur Überwachung industrieller Prozesse.

Im Bereich der für Bioprozesse immer häufiger genutzten Einweg-Technologie (englischer Fachbegriff: *single use technology*) sind Adapter oder Konnektoren bekannt geworden, welche das Einbringen von vorab, z.B. mittels Gammastrahlung sterilisierten, Sensoren in einen, ebenfalls vorab sterilisierten, Einweg-Bioreaktor (englischer Fachbegriff: *single use fermentor*) ermöglichen. Diese Konnektoren sind jedoch für die Anwendung in einem für eine Vielzahl von Prozess-Chargen mehrfach verwendeten Prozessbehälter einer herkömmlichen Prozessanlage, der regelmäßig gereinigt und nach einem der weiter oben beschriebenen SIP-Sterilisationsverfahren sterilisiert wird, häufig nicht akzeptiert bzw. nicht anwendbar.

Die PALL Corporation, Port Washington, USA bietet beispielsweise Konnektoren unter der Bezeichnung "Kleenpak II Sterilkonnektoren" an, die dem Einbringen von Flüssigkeiten oder Sonden bzw. Sensoren in einen Einweg-Prozessbehälter dienen. Diese Konnektoren bestehen aus zwei miteinander verbindbaren Elementen, wobei beide Elemente in ihrem Verbindungsbereich im nicht verbundenen Zustand jeweils mit einem herausziehbaren Streifen verschlossen sind. Die herausziehbaren Streifen bestehen aus Aluminiumfolie mit Polyesterbeschichtung. Zum Einbringen einer Sonde in den Bioprozess kann das erste Element des Konnektors mit dem Prozessbehälter verbunden sein und mit diesem sterilisiert werden, das zweite, die Sonde enthaltende, Element kann mit einer Gamma-Sterilisierung oder einer Autoklavierung steril gemacht werden. Zur Einbringung der Sonde werden die beiden Konnektor-Elemente erst lose miteinander verbunden, danach werden die herausziehbaren Streifen durch seitliches Herausziehen entfernt, anschließend die beiden Elemente dicht miteinander verbunden und abschließend die Sonde in durch das erste Element des Konnektors hindurch in den Prozessbehälter hinein verschoben.

Wesentlicher Nachteil dieser Konnektoren ist, dass die Verbindung zwischen den beiden Elementen nicht mit hoher Sicherheit aseptisch erfolgt, da die beiden Außenflächen der herausziehbaren Streifen der Elemente nicht steril bzw. sterilisierbar sind und somit beim Herausziehen dieser Streifen eine Kontaminationsrisiko gegeben ist. Weiterhin wird das Kontaminationsrisiko dadurch erhöht, dass direkt nach dem Herausziehen der herausziehbaren Streifen die beiden Elemente nicht dicht miteinander verbunden sind, wodurch eine Kontamination durch die nicht sterile Umgebung nicht ausgeschlossen werden kann.

Diese Konnektoren sind auch nicht für mehrfach verwendbare, mit SIP-Verfahren sterilisierbare, Edelstahlprozessbehälter konzipiert.

Es ist somit die Aufgabe der Erfindung, eine Inline-Sensoranordnung anzugeben, die die beschriebenen Nachteile überwindet. Weiter sollen die Nachteile der Inbetriebnahme einer Inline-Sensoranordnung nach dem Stand der Technik überwunden werden. Vorzugsweise soll die Inline-Sensoranordnung universell auch in mehrfach genutzten, reinig- und sterilisierbaren Prozessbehältern einsetzbar sein, und das sichere aseptische Einbringen eines Sensorelements der Inline-Sensoranordnung in einen Prozessbehälter zur Messung der Messgröße in einem in dem Prozessbehälter enthaltenen Medium erlauben. Vorzugsweise soll die Inline-Sensoranordnung dazu geeignet sein, einen Biosensor mit biologischen Erkennungselementen, welche einer Heißdampfsterilisation nicht standhalten, in einen bei hohen Temperaturen zu sterilisierenden Prozessbehälter einzubringen.

Diese Aufgabe wird gelöst durch eine Inline-Sensoranordnung gemäß Anspruch 1, und ein Verfahren zur Herstellung einer Inline-Sensoranordnung gemäß Anspruch 11. Vorteilhafte Ausgestaltungen sind in den abhängigen Ansprüchen angegeben.

Die erfindungsgemäße Inline-Sensoranordnung zur Erfassung von Messwerten einer einen Analytgehalt eines Messmediums repräsentierenden Messgröße umfasst: einen Sensor, welcher dazu ausgestaltet ist, ein mit der Messgröße korreliertes Messsignal zu erzeugen und auszugeben, wobei der Sensor mindestens ein zum Kontakt mit dem Messmedium vorgesehenes steriles Sensorelement aufweist; und ein das mindestens eine Sensorelement umgebendes Gehäuse, das das Sensorelement in einer gegenüber einer Umgebung des Gehäuses dicht verschlossenen Kammer einschließt, und wobei die Kammer in ihrem Innern ein Gasvolumen enthält, das derart ausgestaltet ist, dass ein Einfluss von Schadsubstanzen, insbesondere von reaktiven Stickstoff- und/oder Sauerstoff-Spezies, auf das mindestens eine Sensorelement weitgehend verhindert wird.

Diese Sensoranordnung ermöglicht das aseptische Einbringen von hitzelabilen Sensoren in einen Prozessbehälter, der zuvor hitzesterilisiert wurde, unter Beibehaltung der Funktionalität der Sensoren. Dies wird möglich durch Verwendung der hier und im Folgenden beschriebenen Inline-Sensoranordnung, die ein im Inneren einer dicht verschlossenen, sterilen Kammer befindliches, ebenfalls steriles, Sensorelement beinhaltet bzw. die Sterilisation des Inneren der Kammer einschließlich des Sensorelements mittels Beta- oder Gammastrahlung ermöglicht, die das in der verschlossenen Kammer befindliche Sensorelement vor Schädigung durch eine Hitzesterilisation der Inline-Sensoranordnung von außen, z.B. während die Inline-Sensoranordnung mit dem Prozessbehälter dicht verbunden ist, schützt, und die das aseptische in Kontakt bringen des Sensorelements mit einem in dem Prozessbehälter enthaltenen Medium ermöglicht, indem die Kammer in dem sterilisierten Bereich der Gehäuseaußenseite zum Inneren des Prozessbehälters hin geöffnet wird. Indem die Gehäuseaußenseite, insbesondere ein Bereich der Gehäuseaußenseite, der mit dem Inneren des steril zu haltenden Prozessbehälters in Kontakt gebracht wird oder in Kontakt steht, hitzesterilisiert wird, kann das in der Kammer angeordnete, sterile Sensorelement durch das Öffnen des Gehäuses in diesem Bereich sicher aseptisch mit dem Inneren des Prozessbehälters in Kontakt gebracht und gegebenenfalls in diesen eingebracht werden. Dadurch, dass das analytsensitive, d.h. zur Erfassung einer mit dem Analytgehalt korrelierenden Messgröße ausgestaltete, Sensorelement während der Hitzesterilisierung der Gehäuseaußenseite in der Kammer dicht eingeschlossen ist, ist es mindestens während der Hitzesterilisation vor einem Sterilisierungsmedium, z.B. Heißdampf, das bei der Hitzesterilisierung die Gehäuseaußenseite berührt, geschützt.

Indem das Gasvolumen innerhalb der Kammer derart ausgestaltet ist, dass ein Einfluss von Schadsubstanzen, insbesondere von reaktiven Stickstoff- und/oder Sauerstoff-Spezies, auf das mindestens eine Sensorelement weitgehend verhindert wird, wird eine Schädigung des Sensorelements durch bei einer Sterilisierung des Innern der Kammer einschließlich des Sensorelements mit Beta- oder Gammastrahlung und/oder bei einer Sterilisierung durch Hitzesterilisierung von außen das Sensorelement angreifende reaktive Verbindungen weitgehend unterbunden. Dies ist von besonderem Vorteil, wenn das Sensorelement biologische Erkennungselemente aufweist, die durch solche reaktive Schadsubstanzen irreversibel geschädigt werden können. Es hat sich gezeigt, dass bei Anwesenheit von Sauerstoff und Stickstoff in der Gehäusekammer bei einer Sterilisierung des in der Kammer angeordneten Sensorelements durch Beta- oder Gammastrahlung im Gasvolumen der Kammer reaktive Spezies, z.B. reaktive Sauerstoff- oder Stickstoff-Spezies oder Stickoxide, gebildet werden können, die zu einer Schädigung des Sensorelements führen können. Eine Temperaturerhöhung im Innern der Kammer während der Hitzesterilisierung der Gehäuseaußenseite kann diese Schädigung verstärken.

In einer vorteilhaften Ausgestaltung ist das Gasvolumen im Innern der Kammer weitgehend aus einem Schutzgas, vorzugsweise aus einem oder mehreren Edelgasen, gebildet.

Das Gasvolumen im Innern der Kammer enthält vorteilhaft Sauerstoff und/oder Stickstoff in einem Volumenanteil von jeweils weniger als 1 Volumen-%, bevorzugt jeweils weniger als 0,5 Volumen-%, weiter bevorzugt jeweils weniger als 0,1 Volumen-%.

Im Innern der Kammer können zusätzlich oder alternativ Mittel zur Eliminierung von Sauerstoff und/oder anderer gasförmiger Schadsubstanzen, insbesondere zur Eliminierung oder Adsorption von Stickoxiden und/oder anderen reaktiven Stickstoff- und/oder Sauerstoffspezies angeordnet sein.

Beim Vorgang der Eliminierung wird die Schadsubstanz durch eine chemische Reaktion entfernt. Die Mittel zur Eliminierung von z. B. Sauerstoff basieren auf der Oxidation der entsprechenden Mittel. Diese werden dann häufig auch als Sauerstoff-Absorber bezeichnet. Beim Vorgang der Adsorption wird das Schadgas hingegen durch einen physikalischen Prozess an der Oberfläche der entsprechenden Mittel gebunden bzw. haftet das Schadgas an der Oberfläche an.

Mittel zur Eliminierung können beispielsweise Sauerstoff-Absorber wie Eisen, Ascorbinsäure, fotosensitive Polymere (Handelsname Zero2 der Firma CSIRO, Canberra, Australien) oder Enzyme sein. Eisen kann in Pulverform, ggf. zusammen mit einem halogenid-haltigen Katalysator, offen oder in Behältern, insbesondere mit gasdurchlässiger, ggfs. flexibler Wandung, innerhalb der Kammer vorliegen. Als Absorbenzien kommen weiter nicht-metallische organische Reduktionsmittel (Antioxidationsmittel), z.B. Ascorbinsäure, Ascorbatsalze, Catechol oder Enzymsysteme, z.B. Glukoseoxidase oder Ethanoloxidase in Frage. Zur Eliminierung von Sauerstoff oder sonstigen Schadsubstanzen kommen auch Radikalfänger in Frage.

Weitere Mittel zur Eliminierung von Sauerstoff der sonstigen Schadsubstanzen sind Isoprenoide, Carotinoide, Vitamin E, Provitamin A, Vitamin C, Coenzym Q10, ggfs. synthetisch verestert, Proteine wie Albumine, Katalase, Superoxiddismutase.

Zur Eliminierung von Stickoxid kommt Kaliumpermanganat in Frage. Dieses kann auf einem Trägermaterial, z.B. Aluminiumoxid Al₂O₃, aufgebracht sein.

Mittel zur Adsorption können Adsorbenzien wie Aktivkohle oder Zeolithe sein. Anorganische Schadstoffe können z. B. durch aktiviertes Charcoal Gewebe mit Kupfer-Imprägnierung adsorbiert werden. Dieses ist kommerziell erhältlich unter dem Handelsnamen ACC-Cu, Typ: IAC 442, bei der Firma Infiltec GmbH, Speyer am Rhein, Deutschland.

Zur Eliminierung von Sauerstoff oder Adsorption sauerstoffenthaltenden gasförmigen, reaktiven Substanzen oder anderen Schadsubstanzen wie NOₓ, SOₓ, HCl und NH₃ sind auch folgende kommerziell erhältliche Produkte der Firma Mitsubishi Gas Chemical Company (MGC), Tokio, Japan geeignet, welche sowohl Mittel zur Eliminierung als auch zur Adsorption beinhalten: RP (Revolutionary Preservation)-Agent, Typ RP-K: Mischung aus Aktivkohle, ungesättigten organischen Komponenten, Kieselgur, Polyethylen, Ca(OH)₂ sowie einer saugfähigen Graphitverbindung zur Eliminierung von Sauerstoff und Adsorption, RP-Agent Typ RP-A: Mischung aus ungesättigten organischen Komponenten, Zeolith, Polyethylen, Aktivkohle und CaO

Die Inline-Sensoranordnung ist insbesondere dazu ausgestaltet, ein Sensorelement, insbesondere ein hitzelabiles Sensorelement, aseptisch in einen Prozessbehälter, der zuvor hitzesterilisiert wurde, einzubringen. Die Inline-Sensoranordnung kann hierzu in einer Wand eines Prozessbehälters, beispielsweise mittels einer Armatur oder eines Behälteranschlusses, integriert sein. Durch das Einschließen des Sensorelements in einer gegenüber der Umgebung dicht verschlossenen Gehäusekammer kann das ebenfalls sterile Sensorelement während einer Hitzesterilisierung der mit dem Inneren des Prozessbehälters in Kontakt zu bringenden oder in Kontakt stehenden Gehäuseaußenseite des Gehäuses, die z.B. zusammen mit der Hitzesterilisierung des Inneren des Prozessbehälters erfolgen kann, vor dem Einfluss des Sterilisationsmediums geschützt werden, und so die Funktionalität des Sensorelements im Wesentlichen erhalten werden. Durch die erfindungsgemäße Ausgestaltung des Gasvolumens im Innern der Kammer ist ein Schutz des Sensorelements vor einer Schädigung durch reaktive Sauerstoff- und/oder Stickstoffspezies oder andere Schadsubstanzen, insbesondere während der Hitzesterilisation, ausgeschlossen. Anschließend kann das Sensorelement, wie weiter oben beschrieben, aseptisch durch Öffnen des Gehäuses in einem innerhalb des Prozessbehälters befindlichen und, insbesondere zusammen mit dem Prozessbehälter, hitzesterilisierten Bereich mit dem Inneren des Prozessbehälters bzw. einem darin befindlichen Messmedium in Kontakt gebracht werden.

Um sicherzustellen, dass der Sensor während der Hitzesterilisierung des Gehäuses von außen keiner zu hohen Luftfeuchtigkeit ausgesetzt ist, kann das Gehäuse der Inline-Sensoranordnung derart ausgestaltet und die Kammer gegenüber der Umgebung derart abgedichtet sein, dass während der Hitzesterilisierung des Gehäuses von außen bei einer Temperatur von 110 °C die innerhalb des Gehäuses herrschende relative Feuchte einen Wert von 77 %, bevorzugt von 23 %, weiter bevorzugt von 3 %, noch weiter bevorzugt von 1 % nicht übersteigt.

Die Inline-Sensoranordnung kann mindestens einen Feuchtesensor aufweisen, welcher dazu ausgestaltet ist, eine innerhalb der Kammer herrschende relative Feuchte repräsentierende Messwerte zu erfassen. Die Inline-Sensoranordnung kann weiter dazu ausgestaltet sein, mittels des Feuchtesensors mindestens während der Durchführung einer Hitzesterilisierung der Inline-Sensoranordnung einen Verlauf der die innerhalb der Kammer herrschende relative Feuchte repräsentierenden Messwerte zu erfassen.

Das Gehäuse kann eine aus einer oder mehreren Gehäusekomponenten gebildete Wandung umfassen, die die Kammer dicht einschließt und die eine Barriere gegen das Eindiffundieren von Wasserdampf in die Kammer bildet. Vorteilhaft beträgt eine mittlere Wasserdampfdurchlässigkeit der Wandung, d.h. ein Mittelwert der Wasserdampfdurchlässigkeit der die Wandung bildenden Komponenten, bei einer Temperatur von 110 °C, einer zwischen der Kammer und der Umgebung der Gehäusewandung herrschende Druckdifferenz von weniger als 5 bar und einer Differenz der in der Kammer und in der Umgebung der Wandung herrschenden relativen Feuchte von mehr als 67 % weniger als 420 g/(m²d), bevorzugt von weniger als 125 g/(m²d), weiter bevorzugt von weniger als 15 g/(m²d), noch weiter bevorzugt von weniger als 6 g/(m²d).

Um die Feuchtigkeit in der Kammer gering zu halten, kann die Kammer ein Trocknungsmittel, beispielsweise Silicagel, Kieselgel oder Zeolith, enthalten.

Es ist auch möglich, dass die Inline-Sensoranordnung mindestens eine in die Kammer mündende Zuleitung für ein wasserfreies oder wasserarmes Fluid, insbesondere reinen Stickstoff oder Luft mit einem Wassergehalt von weniger als 50 ppmv (*parts per million by volume*) H₂O, weiter bevorzugt weniger als 5 ppmv H₂O, umfasst. Die Zuleitung kann einen im Strömungsweg des Fluids angeordneten Sterilfilter umfassen. Sie kann mit einem Reservoir verbunden sein, das das Fluid, insbesondere Stickstoff oder Luft, enthält. In die Kammer kann weiter eine Ableitung für das Fluid münden, die vorzugweise ebenfalls einen Sterilfilter umfasst. Die Zu- und die Ableitung sind vorzugsweise außerhalb des Prozessbehälters angeordnet.

Das mindestens eine Sensorelement kann zusätzlich oder alternativ zu den voranstehend beschriebenen Maßnahmen zur Gewährleistung einer geringen relativen Feuchte innerhalb der Kammer mindestens zeitweise von der Umgebung der Gehäuseaußenseite des Gehäuses thermisch entkoppelt oder thermisch entkoppelbar sein. Beispielsweise kann es derart von der Gehäuseaußenseite des Gehäuses thermisch entkoppelt sein, dass während der Einwirkung eines eine Temperatur von 110°C aufweisenden Mediums auf mindestens einen Teilbereich der Gehäuseaußenseite über einen Zeitraum von 15 min die Temperatur des Sensorelements ausgehend von einer Anfangstemperatur des Sensorelements von 25°C bei Beginn dieses Zeitraums um weniger als 55°C, vorzugsweise um weniger als 35°C, weiter bevorzugt um weniger als 10°C, ansteigt. Solche Bedingungen treten beispielsweise bei einer Hitzesterilisierung der Gehäuseaußenseite auf, z.B. im Rahmen eines in einem Prozessbehälter, in den die Inline-Sensoranordnung integriert ist, durchgeführten SIP-Verfahrens. In diesem Fall kann das Medium beispielsweise trockene Heißluft oder Heißdampf sein.

Die Inline-Sensoranordnung kann mit mindestens einem Temperaturfühler/-sensor versehen sein, der dazu ausgestaltet ist, den Temperaturverlauf des mindestens einen Sensorelements, insbesondere während einer Hitzesterilisation mindestens eines Teilbereichs der Gehäuseaußenseite, zu ermitteln.

Die thermische Entkopplung kann beispielsweise durch eine thermische Isolierung oder thermische Dämmung des Sensorelements von der Gehäuseaußenseite erreicht werden, wodurch die pro Zeiteinheit zwischen Gehäuseaußenseite und Sensorelement übertragene Wärmemenge im Vergleich zu einer Ausgestaltung der Inline-Sensoranordnung, bei der keine Isolierung oder Dämmung des Sensorelements von der Gehäuseaußenseite vorgesehen ist, reduziert wird, so dass die Temperaturänderung des Sensorelements entsprechend vermieden oder verlangsamt wird.

Vorteilhaft wird die thermische Entkopplung dadurch erreicht, dass zwischen dem Sensorelement und der Gehäuseaußenseite ein Wärme-Dämmmaterial angeordnet ist und/oder dass mindestens zeitweise in dem Gehäuse ein Druck von weniger als < 100 mbar herrscht. Unter einem Wärme-Dämmmaterial wird hier insbesondere ein homogenes Material niedriger thermischer Leitfähigkeit oder ein mindestens zweiphasiges Material mit gasgefüllten Hohlräumen, insbesondere ein mikroporöses Füllmaterial, verstanden. Das Material niedriger thermischer Leitfähigkeit kann vorteilhaft eine thermische Leitfähigkeit von ≤ 0,5 W m⁻¹ K⁻¹ aufweisen. Das Gehäuse bildet vorteilhaft eine das Sensorelement gasdicht umgebende Kammer.

Um eine thermische Entkopplung des Sensorelements von der Gehäuseaußenseite durch Verringerung des Drucks innerhalb des Gehäuses, insbesondere auf einen Druck von weniger als < 100 mbar, zu ermöglichen, kann das Gehäuse einen in die Kammer mündenden Gasauslass zur Evakuierung aufweisen, welcher gasdicht verschließbar ist. Das Gehäuse bildet in dieser Ausgestaltung bei gasdicht verschlossenem Gasauslass eine das Sensorelement gasdicht umgebende Kammer. Der Gasauslass kann vorteilhaft einen Sterilfilter umfassen. Vorteilhaft ist der Gasauslass außerhalb des Prozessbehälters angeordnet, wenn die Sensoranordnung in dem Prozessbehälter integriert ist.

Der Gasauslass kann in einer Weiterbildung dieser Ausgestaltung in einem, insbesondere reversibel, verschließbaren Konnektor enden, welcher an eine Vakuumpumpe anschließbar ist. Das Gehäuse bzw. die Kammer mit kann daher beispielsweise unmittelbar vor Durchführung der Sterilisation des Prozessbehälters, in den die Inline-Sensoranordnung integriert ist, evakuiert werden. Alternativ ist es auch möglich, eine Inline-Sensoranordnung in eine Wandung des Prozessbehälters zu integrieren, deren Gehäuse bereits evakuiert und anschließend mit inbegriffenem Sensorelement mit einem geeigneten Verfahren sterilisiert worden ist. Unter Evakuieren wird hier insbesondere die Reduzierung des in dem Gehäuse herrschenden Drucks auf einen Wert < 100 mbar verstanden. Vorteilhaft ist es, die Sterilisation des Gehäuses mit inbegriffenem Sensorelement in der Endverpackung mit Gammastrahlen durchzuführen. Zur besseren Sicherstellung, dass in dem Gehäuse zur thermischen Entkopplung von der Umgebung der Gehäuseaußenseite des mindestens einen Sensorelements ein Unterdruck herrscht, kann das Gehäuse mit inbegriffenem Sensorelement bei der Endverpackung mit einem Vakuumiergerät vakuumverpackt werden.

In einer weiteren Ausgestaltung umfasst die Inline-Sensoranordnung einen zur zumindest zeitweisen thermischen Entkopplung des Sensorelements von der Umgebung der Gehäuseaußenseite dienenden Kühler zur zumindest zeitweisen Kühlung mindestens eines Teils der Inline-Sensoranordnung.

Der Kühler kann Mittel zur aktiven und/oder passiven Kühlung des Sensorelements umfassen, wodurch die pro Zeiteinheit zwischen Gehäuseaußenseite und Sensorelement übertragene Wärmemenge zumindest teilweise vom Sensorelement abgeleitet wird, so dass die Temperaturänderung des Sensorelements vermieden oder verlangsamt wird. Diese Mittel können beispielsweise eine Gaskühlung, eine Kühlung mit Kühlflüssigkeit, eine Peltier-Kühlung, Kühlrippen oder eine andere Wärmesenke umfassen.

Der Kühler kann rein passiver Natur sein, beispielsweise kann er eine mit dem Sensorelement in wärmeleitendem Kontakt stehende Wärmesenke umfassen.

Zusätzlich oder alternativ kann der Kühler zur aktiven Kühlung des Sensorelements mindestens einen thermoelektrischen Wandler, z.B. ein Peltier-Element, umfassen. Dieses ist vorteilhaft so angeordnet, dass es eine für die Durchführung von Messungen zum Kontakt mit dem Messmedium bestimmte sensitive Oberfläche des Sensorelements kühlt.

In einer weiteren Ausgestaltung kann der Kühler eine Fluidkühlung umfassen. Diese kann vorteilhaft eine fluiddurchströmbare Kanalstruktur in einem Sensorelementträger, auf dem das Sensorelement angeordnet ist, und/oder in der Gehäusewandung des Gehäuses aufweisen.

Um die thermische Entkopplung zu verbessern, ist es vorteilhaft, wenn das Gehäuse oder mindestens eine oder mehrere das Gehäuse bildende Komponenten aus einem thermisch isolierenden Kunststoff mit einer thermischen Leitfähigkeit von ≤ 0,5 W m⁻¹ K⁻¹, insbesondere aus PEEK, gebildet sind.

Zum Inkontaktbringen des Sensorelements bzw. einer sensitiven Oberfläche des Sensorelements mit einem außerhalb des Gehäuses befindlichen Messmedium kann das Gehäuse einen Wandungsbereich aufweisen, welcher dazu ausgestaltet ist, das Sensorelement in Kontakt mit der Umgebung des Gehäuses zu bringen. Beispielsweise kann der Wandungsbereich dazu ausgestaltet sein, geöffnet zu werden, um eine Verbindung zwischen dem Sensorelement bzw. zwischen der das Sensorelement enthaltenden Kammer und der Umgebung des Gehäuses herzustellen. Dieser Wandungsbereich ist in einem Bereich des Gehäuses angeordnet, der eine mit dem Inneren des Prozessbehälters in Kontakt bringbare oder in Kontakt stehende Gehäuseaußenseite des Gehäuses umfasst.

Das Sensorelement und der Wandungsbereich können relativ zueinander in der Weise beweglich sein, dass das Sensorelement aus dem Gehäuse heraus verschoben werden kann.

In einer Ausgestaltung der Inline-Sensoranordnung, bei der das Sensorelement auf einem Sensorelementträger angeordnet ist, kann der Sensorelementträger relativ zum Gehäuse beweglich gelagert sein, so dass eine Relativbewegung des Sensorelementträgers zum Gehäuse einen Transport des Sensorelements aus dem geöffneten Gehäuse heraus bewirkt.

Beispielsweise kann das Gehäuse, insbesondere die das Sensorelement enthaltende Kammer, einen Wandungsbereich aufweisen, der als Sollbruchstelle ausgebildet ist. Zur Herstellung eines Kontakts zwischen dem Sensorelement und der Gehäuseumgebung kann der Sensorelementträger eine Spitze oder Kante aufweisen, die derart ausgestaltet ist, dass sie bei einer Bewegung des Sensorelementträgers relativ zum Wandungsbereich, welche zu einem Kontakt des Endabschnitts des Sensorelementträgers mit dem Wandungsbereich führt, den Wandungsbereich durchstößt oder aufschneidet. Dieser Wandungsbereich kann beispielsweise als stirnseitige, mit dem Inneren des Prozessbehälters in Kontakt stehende und/oder dem Prozessbehälter zugewandte Wand des Gehäuses ausgestaltet sein, welche beispielsweise durch eine Membran oder Folie gebildet wird.

Alternativ kann das Gehäuse eine Kappe, einen Deckel oder eine Schleusenanordnung umfassen, welche relativ zum Gehäuse beweglich ist, so dass das Gehäuse durch eine Bewegung der Kappe, des Deckels oder der Schleusenanordnung relativ zu einem weiteren Gehäuseteil geöffnet wird, derart dass ein Kontakt zwischen dem Sensorelement und der Umgebung des Gehäuses hergestellt wird. Wenn die Inline-Sensoranordnung im Prozessbehälter integriert ist, stehen die Kappe, der Deckel oder die Schleusenanordnung in Kontakt mit dem Inneren des Prozessbehälters und können bei einer Hitzesterilisation zusammen mit diesem sterilisiert werden, so dass ein Betätigen der Schleusenanordnung nicht dazu führen kann, dass das Innere des Prozessbehälters mit unsterilen Teilen oder mit einer unsterilen Umgebung in Kontakt kommt.

Das Sensorelement kann biologische Erkennungselemente aufweisen. Biologische Erkennungselemente können Proteine wie Enzyme oder Antikörper, DNA-/RNA-Fragmente, Zellorganellen oder ganze Zellen und Mikroorganismen sein. Das biologische Erkennungselement bindet beispielsweise den Analyten spezifisch oder geht eine chemische Reaktion mit dem Analyten ein. Bei dem biologischen Erkennungselement kann es sich beispielsweise um ein unter Beibehaltung von mindestens 10% seiner Aktivität lyophilisierbares Enzym handeln. Beispielsweise kann das Sensorelement als Erkennungselement Glucoseoxidase umfassen.

Beispielsweise kann es sich bei dem Sensor um einen amperometrischen Enzymsensor handeln. Der Sensor kann ein, insbesondere amperometrischer, enzymbasierter Glucosesensor, z.B. mit Glucoseoxidase als Erkennungselement, sein. In Frage kommt hier ein Glucoseoxidase umfassender Glucosesensor, der unter der Bezeichnung B.LV5, B.IV4 von Jobst Technologies GmbH, Freiburg, Deutschland, hergestellt und zum Kauf angeboten wird. Diese amperometrischen, enzymbasierten Sensoren können auch Lactatoxidase als Erkennungselement für Lactatsensoren, Glutamatoxidase für Glutamatsensoren, Glutaminase für Glutaminsensoren umfassen.

Das Gehäuse kann aus Glas gebildet sein und/oder mindestens eine Metall-Lage und/oder eine Lage aus Kunststoff aufweisen und/oder eine Vielzahl von, insbesondere als Diffusionsbarriere für Wasserdampf wirkende, Feststoff-Partikeln, insbesondere Metall-Partikeln, umfassen. Die MetallPartikel können beispielsweise in Form von Sphären oder Plättchen in einen Kunststoff eingebettet sein, der eine Wandung oder eine Dichtung oder eine Klebestelle oder einen Verguss des Gehäuses bildet.

Das Sensorelement kann ein oder mehrere Elektroden umfassen, wobei die Elektroden kontaktierende Leitungen durch einen innerhalb eines Sensorelementträgers, auf dem das Sensorelement angeordnet ist, gebildeten Kanal verlaufen.

Der Sensor kann weiter eine Messschaltung umfassen, die mit den Leitungen verbunden ist und die dazu ausgestaltet ist, ein mit der Messgröße korrelierendes elektrisches Signal zu erfassen. Im Falle, dass der Sensor als amperometrischer Sensor ausgestaltet ist, dient die Messschaltung dazu, zwischen mindestens zwei Elektroden des Sensors eine Spannung anzulegen und den dabei fließenden Strom zu erfassen und diesen oder ein daraus abgeleitetes elektrisches Signal als Messsignal auszugeben. Die Inline-Sensoranordnung kann eine Auswertungsschaltung umfassen, welche dazu ausgestaltet ist, aus den von der Messschaltung ausgegebenen elektrischen Signalen Messwerte der Messgröße in der Einheit der Messgröße zu ermitteln und über eine Schnittstelle an eine übergeordnete Einheit oder über eine Anzeigeeinrichtung, z.B. ein Display, auszugeben.

Die Erfindung betrifft ein Verfahren zur Herstellung einer Inline-Sensoranordnung nach einer der voranstehend beschriebenen Ausgestaltungen. Das Verfahren umfasst die Schritte: Herstellen einer Inline-Sensoranordnung mit einem Sensor, welcher dazu ausgestaltet ist ein mit der Messgröße korreliertes Messsignal zu erzeugen und auszugeben, wobei der Sensor mindestens ein zum Kontakt mit dem Messmedium vorgesehenes Sensorelement aufweist, und mit einem das Sensorelement und mindestens einen Abschnitt des Sensorelements umgebenden Gehäuse, welches das Sensorelement in einer gegenüber einer Umgebung des Gehäuses dicht verschlossenen Kammer einschließt; und Sterilisieren des in der Kammer angeordneten mindestens einen Sensorelements der Inline-Sensoranordnung mittels Bestrahlung durch Beta- oder Gammastrahlung.

Das Herstellen der Inline-Sensoranordnung kann weiter das dichte Verschließen der Kammer umfassen, wobei eine in der Kammer nach dem Verschließen vorliegende relative Feuchte so bemessen ist, dass während einer Hitzesterilisation des Gehäuses von außen bei einer Temperatur von 110 °C über einen Zeitraum von 15 min die innerhalb der Kammer herrschende relative Feuchte einen Wert von 77 %, bevorzugt von 23 %, weiter bevorzugt von 3 %, noch weiter bevorzugt von 1 % nicht übersteigt.

Das beim dichten Verschließen der Kammer in der Kammer vorliegende Gasvolumen wird derart ausgestaltet, dass ein Einfluss von Schadsubstanzen, insbesondere von reaktiven Stickstoff- und/oder Sauerstoff-Spezies, auf das mindestens eine Sensorelement weitgehend verhindert wird. Hierzu kann die Kammer mit Schutzgas befüllt werden, z.B. kann sie vor dem Verschließen auch evakuiert und anschließend das Schutzgas eingeleitet werden. Die Kammer kann auch, ohne zuerst evakuiert zu werden, für eine vorgegebene Zeitdauer mit dem Schutzgas gespült werden. Das Schutzgas ist beispielsweise im Wesentlichen aus einem Edelgas oder einem Gemisch mehrerer Edelgase gebildet. Es ist alternativ oder zusätzlich möglich, dass das Schutzgas Sauerstoff und/oder Stickstoff in einem Volumenanteil von weniger als 1 Volumen-%, bevorzugt weniger als 0,5 Volumen-%, weiter bevorzugt weniger als 0,1 Volumen-% enthält.

Das Verfahren kann weiter das Einbringen eines oder mehrerer Mittel zur Eliminierung von Sauerstoff und/oder anderer gasförmiger Schadsubstanzen, insbesondere zur Eliminierung von Stickoxiden und/oder anderen reaktiven Stickstoff- und/oder Sauerstoffspezies in die Kammer, insbesondere vor dem Verschließen der Kammer, umfassen.

Das Verfahren kann weiter das thermische Entkoppeln des mindestens einen Sensorelements von der Umgebung einer Gehäuseaußenseite des Gehäuses umfassen.

Die Aufgabe wird weiter gelöst durch ein Verfahren zur Inbetriebnahme einer wie oben beschriebenen Inline-Sensoranordnung. Das Verfahren umfasst die folgenden Schritte: Durchführen einer Hitzesterilisierung mindestens eines eine Gehäuseaußenseite des Gehäuses umfassenden Teils der Inline-Sensoranordnung; Öffnen des Gehäuses nach Beendigung der Hitzesterilisierung; und In Kontakt bringen des Sensorelements mit dem Messmedium.

Der einer Hitzesterilisation unterzogene Teil der Inline-Sensoranordnung ist beispielsweise der gesamte mit dem Inneren eines steril zu haltenden Prozessbehälters in Kontakt stehende oder in Kontakt zu bringende Bereich der Inline-Sensoranordnung, insbesondere der gesamte mit dem Inneren des steril zu haltenden Prozessbehälters in Kontakt stehende Bereich der Gehäuseaußenseite des Gehäuses. Indem die Gehäuseaußenseite, insbesondere ein Bereich der Gehäuseaußenseite, der mit dem Inneren des steril zu haltenden Prozessbehälters in Kontakt gebracht wird oder in Kontakt steht, hitzesterilisiert wird, kann das in der Kammer angeordnete, sterile Sensorelement durch das Öffnen des Gehäuses in diesem Bereich sicher aseptisch mit dem Inneren des Prozessbehälters in Kontakt gebracht und gegebenenfalls in diesen eingebracht werden. Dadurch, dass das analytsensitive, d.h. zur Erfassung einer mit dem Analytgehalt korrelierenden Messgröße ausgestaltete, Sensorelement während der Hitzesterilisierung der Gehäuseaußenseite in der Kammer dicht eingeschlossen ist, ist es mindestens während der Hitzesterilisation vor einem Sterilisierungsmedium, z.B. Heißdampf, das bei der Hitzesterilisierung die Gehäuseaußenseite berührt, geschützt. Es ist auch aufgrund der weiter vorn beschriebenen Zusammensetzung des Gasvolumens vor Angriffen durch Schadsubstanzen geschützt. Es hat sich gezeigt, dass biologische Erkennungselemente der oben beschriebenen in der Bioprozessmesstechnik eingesetzten Sensoren zwar bei hoher Luftfeuchtigkeit und hohen Temperaturen, wie sie bei einer Heißdampfsterilisation auftreten, einen Großteil ihrer Funktionalität verlieren können, jedoch behalten sie bei geringer Luftfeuchtigkeit auch bei den bei einer Hitzesterilisierung herrschenden Temperaturen von mindestens 110°C ihre Funktionalität im Wesentlichen bei. Damit kann bereits das dichte Einschließen des Sensorelements in der Kammer zum Erhalt der Funktionalität des Sensorelements trotz hoher Temperaturen bei der Hitzesterilisierung der Gehäuseaußenseite beitragen, so dass das anschließend aseptisch in den Prozessbehälter eingebrachte Sensorelement funktionsfähig ist.

Das Sensorelement kann auf einem Sensorelementträger angeordnet sein, wobei das Gehäuse neben dem Sensorelement mindestens einen Abschnitt des Sensorelementträgers umgibt, so dass mindestens dieser Abschnitt innerhalb der in dem Gehäuse gebildeten Kammer angeordnet ist.

Das Messsignal kann ein elektrisches Signal oder ein optisches Signal sein, das einen Messwert oder eine zeitliche Abfolge von Messwerten der Messgröße repräsentiert.

Zur Inbetriebnahme kann die Inline-Sensoranordnung vor dem Durchführen der Hitzesterilisierung in eine Wandung eines Prozessbehälters integriert werden, und die Hitzesterilisierung der Inline-Sensoranordnung kann in einem einzigen Verfahrensschritt zusammen mit einer Hitzesterilisierung des Prozessbehälters durchgeführt werden, wobei das mit dem Inneren des Prozessbehälters in Kontakt stehende, nun sterilisierte Gehäuse nach Beendigung der Hitzesterilisierung zum Prozessbehälter hin geöffnet wird. Dies kann beispielsweise im Rahmen eines SIP-Verfahrens erfolgen. Die Integration der Inline-Sensoranordnung in die Wandung des Prozessbehälters kann mittels einer Armatur oder eines Prozessanschlusses erfolgen, der mit der Inline-Sensoranordnung dicht, insbesondere fluiddicht, d.h. gas- und/oder flüssigkeitsdicht, verbunden wird. Die Verbindung ist dabei vorzugsweise derart, dass der Prozessbehälter fluiddicht gegenüber der Umgebung des Prozessbehälters verschlossen ist. Dies wird vorzugsweise mittels eines oder mehrerer hygienischer Dichtelemente bewerkstelligt, die derart ausgestaltet sind, dass ihre mit dem Inneren des Prozessbehälters in Kontakt stehenden Flächen mittels eines SIP-Verfahrens sterilisierbar sind. Dieses Dichtelement kann z.B. eine geeignete hygienische Formdichtung sein, wie sie grundsätzlich aus dem Stand der Technik für Armaturen und Wechselarmaturen zum Einsatz in hygienischen Anwendungen bekannt sind.

Das Durchführen der Hitzesterilisierung kann auch in einem Autoklaven erfolgen. Dabei kann die Inline-Sensoranordnung bereits mit dem Prozessbehälter verbunden sein und beide können in den Autoklaven platziert und darin sterilisiert werden.

Zum aseptischen Öffnen des Gehäuses und in Kontakt bringen des Sensorelements mit dem Messmedium kann der mit dem Inneren des Prozessbehälters in Kontakt stehende Bereich des Gehäuses hygienisch, insbesondere kanten-, grat- und spaltfrei, ausgestaltet sein.

Um sicherzustellen, dass der Sensor während der Hitzesterilisierung keiner zu hohen Luftfeuchtigkeit ausgesetzt ist, kann das Gehäuse der Inline-Sensoranordnung derart ausgestaltet und die Kammer gegenüber der Umgebung derart abgedichtet sein, dass während der Hitzesterilisierung des Gehäuses von außen bei einer Temperatur von mindestens 110 °C die innerhalb des Gehäuses herrschende relative Feuchte (auch als relative Luftfeuchte bezeichnet) einen Wert von 77 %, bevorzugt von 23 %, weiter bevorzugt von 3 %, noch weiter bevorzugt von 1 %, nicht übersteigt.

Während der Hitzesterilisierung kann der Verlauf der relativen Feuchte innerhalb der das Sensorelement einschließenden Kammer mittels eines Feuchtesensors der Inline-Sensoranordnung überwacht werden. Der Feuchtesensor kann Bestandteil der Inline-Sensoranordnung sein.

Das Gehäuse kann eine aus einer oder mehreren Gehäusekomponenten gebildete Wandung umfassen, die die Kammer gasdicht einschließt und die eine Barriere gegen das Eindiffundieren von Wasserdampf in die Kammer bildet. Vorteilhaft beträgt eine mittlere Wasserdampfdurchlässigkeit der Gehäusewandung, d.h. ein Mittelwert der Wasserdampfdurchlässigkeit der die Wandung bildenden Komponenten, bei einer Temperatur von 110 °C, einer zwischen der Kammer und der Umgebung der Wandung herrschenden Druckdifferenz von weniger als 5 bar und einer Differenz der in der Kammer und in der Umgebung der Wandung herrschenden relativen Feuchte von mehr als 67 % weniger als 420 g/(m²d), bevorzugt von weniger als 125 g/(m²d), weiter bevorzugt von weniger als 15 g/(m²d), noch weiter bevorzugt von weniger als 6 g/(m²d).

Die Wandung bzw. die die Wandung bildenden Gehäusekomponenten können aus einem Material gebildet sein, durch das Wasserdampf nicht oder nur in einem geringen Maße diffundieren kann, z.B. kann ein solches Material Glas, Kunststoff oder Metall sein. Möglich ist auch die Verwendung eines Kompositmaterials, z.B. eines mehrlagigen Kompositmaterials. Ein für diesen Zweck geeignetes mehrlagiges Kompositmaterial kann beispielsweise mindestens eine Kunststoff- und eine Metalllage oder eine metallisierte Schicht umfassen. Auch mehrlagige Materialien aus verschiedenen Kunststoffmaterialien, z.B. Verbundfolien, sind einsetzbar z. B. PET-PE, PET-PVCD/PE oder PE-EVOH-PE oder Kunststoff-Verbundfolien mit metallischen Schichten wie z.B. ein Verbund aus PET-Aluminium-PE oder Aluminium-PET-Aluminium. Zudem können ein- oder mehrlagige mit Aluminium- oder Siliziumoxid beschichtete Kunststoffmaterialien, Verwendung finden, z.B. PET-SiOₓ/PE.

Das bei dem hier beschriebenen Verfahren zur Inbetriebnahme verwendete Sensorelement kann, wie weiter oben bereits erwähnt, mindestens ein biologisches Erkennungselement für den Analyten aufweisen. Beispielsweise kann es sich bei dem Sensor um einen amperometrischen Enzymsensor handeln. Zum Beispiel kann das Sensorelement als Erkennungselement ein Enzym aufweisen, das unter Beibehaltung von mindestens 10 % seiner Aktivität lyophilisierbar ist. Der Sensor kann ein enzymbasierter Glucosesensor, z.B. mit Glucoseoxidase als Erkennungselement, sein. In Frage kommt hier ein Glucoseoxidase umfassender Glucosesensor, der unter der Bezeichnung B.LV5, B.IV4 von Jobst Technologies GmbH, Freiburg, Deutschland, hergestellt und zum Kauf angeboten wird. Diese amperometrischen, enzymbasierten Sensoren können auch Lactatoxidase als Erkennungselement für Lactatsensoren, Glutamatoxidase für Glutamatsensoren, Glutaminase für Glutaminsensoren umfassen.

Zusätzlich oder alternativ zum Schutz vor zu hoher Luftfeuchtigkeit kann es vorteilhaft sein, das Sensorelement mindestens zeitweise, insbesondere während der Durchführung der Hitzesterilisation, thermisch von der Umgebung des Gehäuses, d.h. der Umgebung einer Gehäuseaußenseite des Gehäuses, zu entkoppeln. Das Sensorelement kann insbesondere während des voranstehend beschriebenen Verfahrens zur Inbetriebnahme bis nach der Beendigung der Hitzesterilisierung thermisch von der Umgebung des Gehäuses entkoppelt sein. Vorteilhaft wird das Sensorelement dabei thermisch derart von der Umgebung einer Gehäuseaußenseite des Gehäuses thermisch entkoppelt, dass während der Hitzesterilisierung der Inline-Sensoranordnung die Temperatur des Sensorelements auf weniger als 80 °C, vorzugsweise auf weniger als 50 °C, weiter bevorzugt auf weniger als 35 °C ansteigt. Dies kann zusätzlich oder alternativ zur Gewährleistung einer geringen relativen Feuchte innerhalb der Kammer dazu dienen, eine Beeinträchtigung der Messeigenschaften des Sensorelements, insbesondere soweit es biologische Erkennungselemente umfasst, zu vermeiden. Zum Zweck der mindestens zeitweisen thermischen Entkopplung des Sensorelement von der Umgebung des Gehäuses kann das Sensorelement, beispielsweise während einer Hitzesterilisierung, vom zu sterilisierenden Volumen des Prozessbehälters und von den vom Sterilisationsmedium berührten Teilen beabstandet angeordnet werden. Beispielsweise kann der Prozessbehälter einen Anschluss aufweisen, der einen mit dem Prozessbehälter kommunizierenden Anschlussraum umgibt, und der vor dem Durchführen der Hitzesterilisierung mit einem zu dem Anschluss komplementären Prozessanschluss der Inline-Sensoranordnung verbunden wird. Der Prozessanschluss wird dabei derart mit dem Gehäuse der Inline-Sensoranordnung verbunden, dass das Sensorelement auf einer von dem Prozessbehälter abgewandten Seite außerhalb des Anschlussraums angeordnet ist, wenn der Prozessanschluss und der Anschluss des Prozessbehälters miteinander verbunden sind. Beispielsweise kann eine Stirnfläche des Gehäuses der Inline-Sensoranordnung den Anschlussraum verschließen, wenn der Anschluss des Prozessbehälters mit dem Prozessanschluss der Inline-Sensoranordnung verbunden ist. In diesem Fall kommt nur die Stirnfläche des Gehäuses mit dem Sterilisationsmedium in Berührung und wird von diesem erhitzt, während das Sensorelement hinter der Stirnfläche in einem Abstand von der Stirnfläche angeordnet ist und somit geringeren Temperaturen ausgesetzt ist. Auf diese Weise kann eine thermische Entkopplung des Sensorelements von dem Inneren des Prozessbehälters, der eine Hitzesterilisation ausgesetzt werden kann, erreicht werden.

Vorteilhaft kann die Inline-Sensoranordnung bei ihrer Inbetriebnahme vor der Hitzesterilisierung gekühlt werden, vorzugsweise auf weniger als 8°C, weiter bevorzugt auf weniger als -13°C, weiter bevorzugt auf weniger als -20°C. Während der Hitzesterilisierung kann der Temperaturverlauf des mindestens einen Sensorelements durch mindestens einen Temperaturfühler/-sensor der Inline-Sensoranordnung überwacht werden.

Die Erfindung wird im Folgenden anhand der in den Figuren dargestellten Ausführungsbeispiele näher erläutert. Es zeigen:
- Fig. 1: eine schematische Darstellung eines ersten Ausführungsbeispiels einer in die Wandung eines Prozessbehälters integrierten Inline-Sensoranordnung;
- Fig. 2: eine schematische Darstellung eines zweiten Ausführungsbeispiels einer in die Wandung eines Prozessbehälters integrierten Inline-Sensoranordnung;
- Fig. 3: eine schematische Darstellung eines dritten Ausführungsbeispiels einer in die Wandung eines Prozessbehälters integrierten Inline-Sensoranordnung;
- Fig. 4: eine schematische Darstellung eines Prozessanschlusses einer Inline-Sensoranordnung, der mit einem Anschluss eines Prozessbehälters verbunden ist.

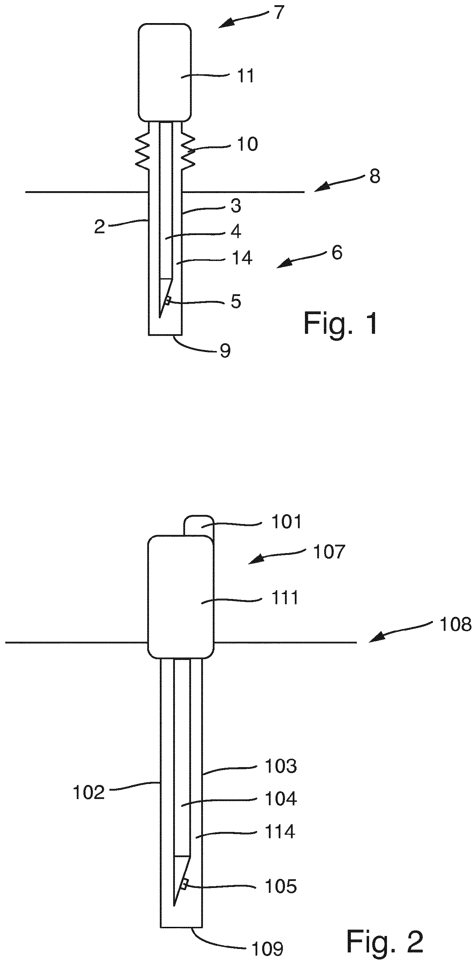

In Fig. 1 ist schematisch eine Inline-Sensoranordnung 7 dargestellt, die in die Wandung eines Prozessbehälters 8, z.B. eine Rohrleitung oder einen Fermenter, integriert ist. In dem Prozessbehälter 8 wird ein biotechnologischer, vor Kontamination zu schützender Prozess durchgeführt. Zur Integration der Inline-Sensoranordnung 1 in den Prozessbehälter 8 kann die Inline-Sensoranordnung 7 einen Prozessanschluss (in Fig. 1 nicht dargestellt) aufweisen, der mit einem zu dem Prozessanschluss komplementären Anschluss des Prozessbehälters 8 fluiddicht verbunden ist. Alternativ kann die Inline-Sensoranordnung 1 in einer Armatur angeordnet sein, die fluiddicht an einem Anschluss des Prozessbehälters 8 befestigt ist.

Die Inline-Sensoranordnung 1 umfasst einen Sensor, der im Wesentlichen durch ein analytsensitives Sensorelement 5 und eine mit dem Sensorelement 5 verbundenen Messschaltung gebildet wird. Das analytsensitive Sensorelement 5 ist dazu bestimmt, zur Messung einer Analyse-Messgröße eines Messmediums mit diesem in Kontakt gebracht zu werden. Das Sensorelement 5 kann beispielsweise eine oder mehrere Elektroden aufweisen, welche mit biologischen Erkennungselementen modifiziert sind. Die Erkennungselemente können beispielsweise an der Elektrodenoberfläche immobilisierte, den Analyten spezifisch bindende Verbindungen umfassen. Als spezifisch bindende Verbindungen kommen z.B. Enzyme oder Proteine in Frage. Das Sensorelement 5 ist auf einem Sensorelementträger 4 angeordnet. Der Sensorelementträger 4 ist im hier gezeigten Beispiel stabförmig ausgestaltet. Innerhalb des stabförmigen Sensorelementträgers 4 kann ein Hohlraum, z.B. ein sich in axialer Richtung erstreckender Kanal, gebildet sein, durch den das Sensorelement 6 elektrisch kontaktierende Ableitungen geführt sind (hier nicht dargestellt).

An einem prozessabgewandten Ende weist die Inline-Sensoranordnung 7 ein Elektronikgehäuse 11 auf, in dem die der Erfassung von Messwerten dienende Messschaltung angeordnet ist. Die Messschaltung ist mit den durch den Sensorelementträger 4 geführten Ableitungen elektrisch leitend verbunden und dazu ausgestaltet, mit der zu erfassenden Messgröße korrelierende elektrische Messsignale zu erzeugen. Im hier gezeigten Beispiel ist der Sensor der Inline-Sensoranordnung 7 als amperometrischer Enzymsensor ausgestaltet. In diesem Fall ist die Messschaltung dazu ausgestaltet, eine Spannung zwischen zwei Elektroden des Sensorelements 5 anzulegen oder einzuregeln und den dabei durch ein beide Elektroden kontaktierendes Messmedium fließenden Strom zu erfassen. Die Messschaltung gibt den erfassten Strom oder einen daraus abgeleiteten, insbesondere digitalen, Wert als Messsignal aus. Die Messschaltung kann mit einer übergeordneten Auswerte- oder Kontrolleinheit (nicht dargestellt) verbunden werden, die die von der Messschaltung ausgegebenen Messsignale empfängt und weiterverarbeitet. Das Elektronikgehäuse 10 kann zur Verbindung mit der übergeordneten Auswerte- oder Kontrolleinheit eine, beispielsweise eine Primärseite einer Steckverbindung umfassende, Schnittstelle aufweisen. Die Auswerte- oder Kontrolleinheit kann über ein Kabel, das die Sekundärseite der Steckverbindung umfasst, mit der Inline-Sensoranordnung 7 verbunden sein.

Die Inline-Sensoranordnung 7 umfasst ein zusätzliches Gehäuse 2, das einen in den Prozessbehälter ragenden, das Sensorelement 5 umfassenden, Abschnitt des Sensorelementträgers 4, umgibt. Im hier gezeigten Beispiel umfasst das Gehäuse 2 mehrere Gehäusekomponenten, nämlich einen rohrförmigen Schaft 3, der an einem in den Prozessbehälter 8 hinein ragenden Ende durch eine stirnseitige Wand 9 verschlossen ist, und einen am der Wand gegenüberliegende Ende des Schafts 3 mit diesem verbundenen Faltenbalg 10. Die stirnseitige Wand 9 kann durch eine Metall-KunststoffVerbund-Folie gebildet sein, die stoffschlüssig, z.B. mittels eines Klebstoffs oder Vergusses mit dem rohrförmigen Schaft 3 verbunden ist. Auf seiner der Wand 9 gegenüberliegenden Seite ist das Gehäuse 2 durch einen Verguss (nicht eingezeichnet) dicht verschlossen und mit dem Elektronikgehäuse 11 verbunden.

Das Gehäuse 2 schließt den vorderen, das Sensorelement 5 umfassenden Abschnitt des Sensorelementträgers 4 vollständig in einer gasdichten Kammer 14 ein, so dass keine Verbindung zwischen dem in dem Gehäuse 2 eingeschlossenen Volumen der Kammer 14 und dem Inneren des Prozessbehälters 7 besteht. Die gasdichte Kammer 14 enthält ein Gasvolumen, das derart ausgestaltet ist, dass ein Einfluss von Schadsubstanzen, insbesondere von reaktiven Stickstoff- und/oder Sauerstoff-Spezies, auf das mindestens eine Sensorelement weitgehend verhindert wird. Im vorliegenden Beispiel enthält das Gasvolumen Sauerstoff und/oder Stickstoff in einem Volumenanteil von weniger als 1 Volumen-%, bevorzugt weniger als 0,5 Volumen-%, weiter bevorzugt weniger als 0,1 Volumen-%. Die in der Kammer 14 vorliegende Luftfeuchtigkeit ist so bemessen, dass bei einer Temperatur von 110°C die in der Kammer enthaltene relative Feuchte weniger als 77 % beträgt. Zusätzlich kann in der Kammer ein Trockenmittel, z.B. Silicagel, Zeolith, Kieselgel o.ä. enthalten sein, um die relative Feuchte in der Kammer 14 weiter herabzusetzen.

Das Gehäuse 2 bildet eine Barriere für die Eindiffusion von Wasser bzw. Wasserdampf aus der Umgebung in die Kammer. Die mittlere Wasserdampfdurchlässigkeit des im vorliegenden Beispiel aus mehreren Gehäusekomponenten aus unterschiedlichen Materialien gebildeten Gehäuses 2 beträgt bei einer Temperatur von 110 °C und einer Druckdifferenz zwischen der Umgebung und der Kammer 14 kleiner 5 bar und einer Differenz der relativen Feuchten innerhalb der Kammer 14 und der Umgebung kleiner als 420 g/m²d oder vorzugweise noch geringer. Die verschiedenen Materialien der Komponenten des Gehäuses 2 sind so zusammengestellt, dass die Wasserdampfdurchlässigkeit des Gehäuses 2 im Mittel unter diesem Wert liegt. In Frage kommen beispielsweise PPSU, ECTFE, PEEK, PPS, PFA oder PCTFE.

In Frage kommen als Materialien für den rohrförmigen Schaft 3 Glas, Metall oder Kunststoffe, welche eine entsprechend geringe Wasserdampfdurchlässigkeit aufweisen. Dies sind beispielsweise Metalle, oder wasserdampfundurchlässige Kunststoffe.

In Frage kommen als Materialien für eine Gehäusewandung, insbesondere für den rohrförmigen Schaft oder die stirnseitige Wand 9 außerdem Kompositmaterialien wie z.B. mehrlagige Materialien, die mindestens eine Lage aus einem Material, das eine hohe Barriere gegen die Eindiffusion von Wasser aus der Umgebung bildet, umfassen. Beispielsweise kann das Kompositmaterial eine Folie mit einer Lage aus Metall, z.B. Aluminium, und/oder einer Lage aus einem Barriere-Kunststoff umfassen. Beispielsweise kann ein solches Kompositmaterial ein metallbeschichteter Kunststoff sein. Ein für den Schaft 3 oder die stirnseitigen Wand 9 geeignetes Kompositmaterial kann außerdem statt einer durchgehenden Lage des Materials mit hoher Barrierewirkung auch eine Vielzahl von in einem Basismaterial, z.B. einem Kunststoff, eingebetteten Partikeln aus einem solchen Barrierematerial umfassen. Die eingebetteten Partikel können beispielsweise Metallpartikel sein.

Die Kammer 14 abdichtende Vergüsse, Klebstoffe oder Dichtungen können ebenfalls Materialien mit hoher Barrierewirkung sein. Insbesondere können sie aus einem Barrierekunststoff gebildet sein oder sie können aus Kompositmaterialien gebildet sein, z.B. aus einem Feststoffpartikel umfassenden Polymer.

Es ist möglich, dass ein Verguss oder eine Dichtung, die verglichen mit dem Schaft 3 und der stirnseitigen Wand 9 nur einen geringen Teil der die Kammer 14 begrenzenden Wandung ausmacht, aus einem konventionell für Flüssigkeits-Analysesensoren genutzten Verguss- oder Dichtungsmaterial gebildet sind. Weisen diese Materialien eine geringe Barrierewirkung für Wasserdampf auf, kann dies dadurch ausgeglichen werden, dass für den Schaft 3 oder die Wand 9 ein Material mit sehr geringer Wasserdampfdurchlässigkeit gewählt wird, so dass die Wasserdampfdurchlässigkeit aller die Kammer 14 einschließenden Komponenten des Gehäuses im Mittel unter dem oben genannten Grenzwert bleibt.

Die Wasserdampfdurchlässigkeit eines Materials wird in der Einheit g/m²d angegeben. Sie wird beispielsweise anhand der DIN 53122-1 / DIN 53122-A gravimetrisch bestimmt, indem ein mit einem Trockenmittel gefüllter Prüfbehälter durch eine Probe aus dem zu untersuchenden Material verschlossen wird und einem definierten Prüfklima ausgesetzt wird. Die durch die Probe permeierende Wassermenge wird durch Wiegen bestimmt. Verwandte Normen sind ISO 2528:1995, ASTM E-96.

Vor Beginn der Durchführung eines biotechnologischen Prozesses, welcher unter sterilen bzw. aseptischen Bedingungen durchgeführt wird, in dem Prozessbehälter 8 kann die Inline-Sensoranordnung 7 in der Gehäusewandung des Prozessbehälters 8 dicht verbunden integriert werden. Die Kammer 14 sowie der Sensorträger 4 und das Sensorelement 5 sind zu diesem Zeitpunkt bereits steril. Eine Sterilisierung der Kammer 14 und der darin angeordneten Elemente kann beispielsweise mittels Bestrahlung mit Gammastrahlung erfolgen. Die Sterilisierung kann vorteilhaft bereits vom Hersteller bei der Herstellung des Sensorelements 5 bzw. der Inline-Sensoranordnung 7 durchgeführt werden.

Die Inbetriebnahme der Inline-Sensoranordnung 7 erfolgt in folgender Weise:
In einem ersten Schritt wird der Prozessbehälter 8 zusammen mit demjenigen Bereich der Außenseite des Gehäuses 2 der integrierten Inline-Sensoranordnung 7 hitzesterilisiert, der mit dem Prozessbehälter 8 in Kontakt steht, beispielsweise mittels einer Heißdampfsterilisation. Der Heißdampf wirkt dabei nur auf die Außenseite des Gehäuses 2 ein, die mit dem Inneren des Prozessbehälters in Kontakt steht. Ein typischer Temperaturverlauf der dem Heißdampf ausgesetzten Gehäuseaußenseite umfasst eine Aufheizphase von einer Anfangstemperatur, z.B. Zimmertemperatur (ca. 25 °C), auf 140°C über einen Zeitraum von 1 h, eine Phase von beispielsweise 1 h Länge, während derer die Temperatur auf 140°C gehalten wird und eine anschließende Abkühlphase, während derer über einen Zeitraum von beispielsweise 4 h das Gehäuse wieder auf Zimmertemperatur abgekühlt wird. Um eine vollständige Sterilisierung zu erreichen, sind Dichtelemente, die die Verbindung der Inline-Sensoranordnung 7 mit dem Prozessbehälter 8 abdichten, hygienisch ausgestaltet, d.h. ihre mit dem Inneren 6 des Prozessbehälter 8 in Kontakt stehenden Oberflächenbereiche sind vollständig für das Sterilisationsmedium, im vorliegenden Beispiel Heißdampf, zugänglich und sterilisierbar. Auch die mit dem Inneren des Prozessbehälters 8 in Kontakt stehende Gehäuseaußenseite des Gehäuses 2 ist hygienisch ausgestaltet, d.h. sie weist keine Spalte oder Grate oder Kanten auf, die nicht vollständig für das Sterilisationsmedium zugänglich sind und damit sterilisierbar sind.

Nach Beendigung der Sterilisation und nach dem Abkühlen des Prozesses auf Temperaturen von weniger als 80°C, bevorzugt weniger als 60°C oder gar als 40°C, wird ein Kontakt zwischen dem Sensorelement 5 bzw. der Kammer 14 und dem Inneren 6 des Prozessbehälters 8 hergestellt, um das Erfassen von Messwerten in einem in dem Prozessbehälter 8 enthaltenen oder durch den Prozessbehälter 8 strömenden Messmedium zu ermöglichen.

Im vorliegenden Beispiel ist die dem Prozessbehälter 8 zugewandte und mit dessen Inneren 6 in Kontakt stehende, stirnseitige Wand 9 des Gehäuses 4 so dünn ausgestaltet, dass sie durch eine mechanische Krafteinwirkung durchstoßen werden kann. Das dieser Wand 9 zugewandte Ende des Sensorelementträgers 4 mit dem Sensorelement 5 weist eine Spitze oder Kante auf. Der Sensorelementträger 4 ist axial beweglich gelagert, im hier gezeigten Beispiel mittels eines als Faltenbalg 10 ausgestalteten Wandungsbereichs des Gehäuses 2. Hier und in der folgenden Beschreibung weiterer Ausführungsbeispiele wird der Begriff "axial" mit Bezug auf eine Zylindersymmetrieachse eines Sensorelementträgers oder eines rohrförmigen Gehäuseschafts der Inline-Sensoranordnung verwendet. Der Faltenbalg 10 kann in der Weise kontrahiert werden, dass die Differenz zwischen der (in axialer Richtung gemessenen) Länge des Gehäuses 104 bei entspanntem Zustand des Faltenbalgs 10 und der Länge des Gehäuses 2 bei maximal kontrahiertem Faltenbalg 10 größer ist als der Abstand zwischen der stirnseitigen Wand 9 und dem Sensorelement 5, wobei dieser Abstand einer in axialer Richtung zwischen der Wand 9 und dem am weitesten von der Wand 9 entfernt angeordneten Punkt des Sensorelements 5 verlaufenden Strecke entspricht. Die Inline-Sensoranordnung 7 kann zusätzlich (in Fig. 1 nicht dargestellte) Arretierungselemente aufweisen, die den Faltenbalg in kontrahierter Stellung fixieren. Wird der Faltenbalg 10 kontrahiert, durchstößt das Sensorelement 5 somit die Wand 9 und ragt über das frontseitige Ende des Gehäuses 2 hinaus. Auf diese Weise wird die Kammer 14 zum Inneren 6 des Prozessbehälters 8 hin geöffnet und das Sensorelement wird in Kontakt mit einem in dem Prozessbehälter 8 enthaltenen oder diesen durchströmenden Prozessmedium in Kontakt gebracht. Die Herstellung des Kontakts zwischen dem Sensorelement 5 und dem Inneren 6 des Prozessbehälters 8 erfolgt dabei aseptisch, da das Sensorelement 5 und das Innere der Kammer 14 vor dem Öffnen bereits sterilisiert wurden. Es ergibt sich auch beim Öffnen der Wand 9 keine Möglichkeit eines Kontakts mit der unsterilen Umgebung des Prozessbehälters oder mit unsterilen Teilen der Inline-Sensoranordnung 7. In der kontrahierten Stellung des Faltenbalgs 10 kann die Inline-Sensoranordnung 7 dazu dienen, die zu erfassende Messgröße des in dem Prozessbehälter 8 enthaltenen oder diesen durchströmenden Messmediums zu überwachen.

Alternativ kann die Inline-Sensoranordnung 7 bei der Inbetriebnahme zusammen mit dem Prozessbehälter 8 in einem Autoklaven einer Hitzesterilisation unterzogen werden. Der sterilisierte Prozessbehälter 8 kann zusammen mit der sterilisierten Inline-Sensoranordnung 7 anschließend in einer biotechnologischen Anlage eingebaut und zur Durchführung eines biotechnologischen Prozesses verwendet werden. Die aseptische Einführung des Sensorelements in den Prozessbehälter erfolgt in dieser Ausgestaltung in gleicher Weise wie voranstehend beschrieben. Insbesondere ist auch hier ein Kontakt mit unsterilen Teilen oder der unsterilen Umgebung ausgeschlossen.

Nach dem Abschluss des biotechnologischen Prozesses wird der Sensor verworfen, da eine erneute Sterilisierung des Prozessbehälters 8 mit der Inline-Sensoranordnung 7 gemäß dem hier beschriebenen Ausführungsbeispiel, bei der das Gehäuse 2 bei Inbetriebnahme irreversibel zerstört wird, nicht möglich ist. Wird der Prozessbehälter 8 erneut verwendet, um einen neuen Bioprozess durchzuführen, wird zunächst die Inline-Sensoranordnung 7 gegen eine noch nicht verwendete, gleichartige Inline-Sensoranordnung 7 mit einem intakten Gehäuse 2 ausgetauscht.

Weisen die die Kammer 14 einschließenden Komponenten des Gehäuses 2 der Inline-Sensoranordnung 7 im Mittel eine Wasserdampfdurchlässigkeit von weniger als 420 g/(m²d), vorzugsweise von weniger als 125 g/(m²d), weiter bevorzugt von weniger als 15 g/(m²d) oder sogar von weniger als 6 g/(m²d) auf, so dringt in die Kammer 14 während der Hitzesterilisierung z.B. mit Heißdampf so wenig Wasserdampf ein, dass die relative Feuchte innerhalb der Kammer 14 über die gesamte Dauer der Hitzesterilisierung nicht über einen Wert von 77 % steigt oder sogar deutlich geringer, z.B. unterhalb von 23% oder sogar unterhalb von 3 % liegt. Die relative Feuchte kann sogar bei geeigneter Materialwahl des Gehäuses bei unter 1 % bleiben. Solche Werte können auch dann erreicht werden, wenn die relative Feuchte der beim Herstellen der Inline-Sensoranordnung 7 in die Kammer 14 eingeschlossenen Luft bei Zimmertemperatur (25°C) bis zu ca. 30 % beträgt. Es hat sich gezeigt, dass bei diesen Bedingungen trotz der bei der Hitzesterilisierung auftretenden hohen Temperaturen, beispielsweise gemäß dem oben angegebenen Temperaturverlauf, keine Zerstörung der biologischen Erkennungselemente auftritt. Dies konnte beispielsweise für Sensorelemente enzym-basierter Glucose-Sensoren, die Glucoseoxidase als biologische Erkennungselemente umfassen, beispielsweise die unter der Bezeichnung B.LV5, B.IV4 von Jobst Technologies GmbH, Freiburg, Deutschland, hergestellten und zum Verkauf angebotenen Glucose-Sensoren, nachgewiesen werden.

Das Gehäuse 2 kann vorteilhaft einen Außendurchmesser von ca. 12 mm aufweisen. Viele Standard-Armaturen, die in der Prozessmesstechnik zur Integration von Sensoren in die Wandung von Prozessbehältnissen verwendet werden, sind dazu ausgestaltet, stabförmige Sensoren mit einem Außendurchmesser von 12 mm aufzunehmen. Weist das Gehäuse 2 einen Außendurchmesser von 12 mm auf, kann es ohne weiteres mittels solcher herkömmlicher Armaturen in die Wandung 9 des Prozessbehälters 8 integriert werden.

Die Herstellung der Inline-Sensoranordnung 7 kann so erfolgen, dass der Sensorelementträger 4 mit dem darauf angeordneten Sensorelement 5 in den bereits mit der Wandung 9 und dem Faltenbalg 10 fest verbundenen rohrförmigen Schaft 3 eingeführt wird, während das Gehäuse 2 auf seiner der Wand 9 gegenüberliegenden Seite noch offen ist. In einem weiteren Schritt kann das Gehäuse 2 dann auf dieser Seite durch einen Verguss unter Bildung der den Sensorelementträger 4 und das Sensorelement 5 umgebenden Kammer 14 verschlossen werden, wobei mindestens die das Sensorelement 5 kontaktierenden elektrischen Leitungen durch den Verguss hindurchgeführt werden, um außerhalb der Kammer 14 mit einer Messschaltung verbunden zu werden. Die die Messschaltung umfassende Platine wird in dem Elektronikgehäuse 11 angeordnet, das an dem Gehäuse 2 fixiert wird.

Vor dem Verschließen der Kammer 14 kann die Luftfeuchtigkeit in der Kammer so eingestellt werden, dass die relative Feuchte innerhalb der Kammer 14 bei einer Temperatur von 110 °C unterhalb der oben genannten Grenzwerte bleibt. Beispielsweise kann hierzu ein getrocknetes Gas in der Kammer 14 eingeschlossen werden und/oder ein Trockenmittel in die Kammer 14 gegeben werden. Die Herstellung kann außerdem die Sterilisierung des Inneren der Kammer 14 einschließlich des Sensorträgers 4 und des Sensorelements 5 mittels Gammastrahlung umfassen. Alternativ kann dies kurz vor der Inbetriebnahme der Inline-Sensoranordnung 7 seitens des Betreibers der Anlage, in der der mittels der Inline-Sensoranordnung 7 zu überwachende biotechnologische Prozess durchgeführt wird, erfolgen.

Fig. 2 zeigt eine schematische Darstellung eines weiteren Ausführungsbeispiels einer Inline-Sensoranordnung 107, die in die Wandung eines Prozessbehälters 108 integriert ist. Bei dem Prozessbehälter kann es sich z.B. um eine Rohrleitung oder einen Fermenter handeln, der aus einem für den in dem Prozessbehälter 108 durchgeführten, beispielsweise biotechnologischen, Prozess geeigneten Material, z.B. Edelstahl, gebildet ist. Die Inline-Sensoranordnung 107 erlaubt eine thermische Entkopplung eines Sensorelements 105 mit biologischen Erkennungselementen von einer Hitzesterilisation ausgesetzten Komponenten, z.B. einer Gehäuseaußenseite, der Inline-Sensoranordnung 107. Auf diese Weise kann gewährleistet werden, dass auch bei einer Sterilisierung der Inline-Sensoranordnung 107 mittels einer Hitzesterilisation die biologischen Erkennungselemente keinen Temperaturen ausgesetzt werden, die zu einer Denaturierung der Erkennungselemente und damit zu einer Beeinträchtigung der Funktionalität des Sensorelements 105 führen.
Die Inline-Sensoranordnung 107 umfasst einen Sensor, der im Wesentlichen durch ein analytsensitives Sensorelement 105 und eine mit dem Sensorelement 105 verbundene Messschaltung gebildet ist. Das Sensorelement 105 ist dazu bestimmt, zur Messung einer Analyse-Messgröße eines in dem Prozessbehälter 108 enthaltenen Messmediums mit diesem in Kontakt gebracht zu werden. Es kann beispielsweise eine oder mehrere Elektroden aufweisen, welche mit biologischen Erkennungselementen, beispielsweise den Analyten spezifisch bindende Substanzen wie Enzyme oder Proteine, modifiziert sind. Das Sensorelement 105 ist auf einem stabförmigen Sensorelementträger 104 angeordnet. Innerhalb des stabförmigen Sensorelementträgers 104 kann ein Hohlraum, z.B. ein sich in axialer Richtung erstreckender Kanal, gebildet sein, durch den das Sensorelement 106 elektrisch kontaktierende Ableitungen geführt sind (hier nicht dargestellt).

An ihrem prozessabgewandten Ende weist die Inline-Sensoranordnung 107 ein Elektronikgehäuse 111 auf, in dem die der Erfassung von Messwerten dienende Messschaltung angeordnet ist. Die Messschaltung ist mit den durch den Sensorelementträger 4 geführten Ableitungen elektrisch leitend verbunden und dazu ausgestaltet, mit der zu erfassenden Messgröße korrelierende elektrische Messsignale zu erzeugen. Die Messschaltung kann analog ausgestaltet sein wie die Messschaltung des in Fig. 1 dargestellten Ausführungsbeispiels. Sie kann ebenfalls mit einer übergeordneten Auswerte- oder Kontrolleinheit verbunden werden, die die von der Messschaltung ausgegebenen Messsignale empfängt und weiterverarbeitet. Das Elektronikgehäuse 111 kann zur Verbindung mit der übergeordneten Auswerte- oder Kontrolleinheit eine, beispielsweise eine Primärseite einer Steckverbindung umfassende, Schnittstelle aufweisen. Die Auswerte- oder Kontrolleinheit kann über ein Kabel, das die Sekundärseite der Steckverbindung umfasst, mit der Inline-Sensoranordnung 107 verbunden sein.

Die Inline-Sensoranordnung 107 umfasst ein zusätzliches Gehäuse 102, das einen in den Prozessbehälter 108 ragenden, das Sensorelement 105 umfassenden, Abschnitt des Sensorelementträgers 104, umgibt und in einer gegenüber der Umgebung der Inline-Sensoranordnung, insbesondere gegenüber dem Inneren des Prozessbehälters 108 gasdicht verschlossenen, Kammer 114 einschließt. Im hier gezeigten Beispiel weist das Gehäuse 102 einen rohrförmigen Schaft 103 und eine den rohrförmigen Schaft 103 stirnseitig verschließende Wand 109 auf. Auf der der Wand 109 gegenüberliegenden Seite ist der rohrförmige Schaft durch einen Verguss verschlossen und abgedichtet. Die in dem Gehäuse 102 eingeschlossene Kammer 114 kann ein Gas, z.B. Stickstoff, oder ein Gasgemisch, beispielsweise Luft enthalten.

Durch das Elektronikgehäuse 111 ist eine Gasleitung geführt, deren erstes Ende ins Innere des Gehäuses 102, genauer in die Kammer 114, mündet und deren zweites Ende einen Konnektor 101 aufweist. Der Konnektor 101 kann am Elektronikgehäuse 111 befestigt sein. Vorteilhaft kann der Konnektor 101 als Sterilkonnektor ausgestaltet sein. Um den innerhalb des Gehäuses 102 herrschenden Druck zu verringern, kann der Konnektor 101 mit einer Vakuumpumpe verbunden werden. Die Evakuierung des Gehäuses 102 bzw. die Absenkung des in dem Gehäuse 102 herrschenden Drucks auf einen Wert von weniger als 100 mbar dient der thermischen Entkopplung des auf dem Sensorelementträger 104 angeordneten Sensorelements 105 von der Außenseite des Gehäuses 102, d.h. der zur Umgebung des Gehäuses 102 hin weisenden Außenfläche der Gehäusewandung bzw. von der Umgebung der Außenseite des Gehäuses 102. Die Innenseite, d.h. die zur Kammer 114 hin weisende Gehäuseinnenwandung des Gehäuses 102 kann als weitere Maßnahme zur thermischen Entkopplung verspiegelt sein.

Zur Inbetriebnahme der Inline-Sensoranordnung 107 kann diese vor Beginn der Durchführung eines Bioprozesses in dem Prozessbehälter 108 in dessen Gehäusewandung integriert werden. Die Kammer 114 sowie der Sensorträger 104 und das Sensorelement 105 sind zu diesem Zeitpunkt vorteilhaft bereits steril. Vorteilhaft ist das Sensorelement 105 zu diesem Zeitpunkt auch bereits von der Umgebung der Gehäuseaußenseite oder nach außen gerichteten Gehäusewandungsfläche thermisch entkoppelt. Eine Sterilisierung des Inneren des Gehäuses 102 und der darin angeordneten Elemente kann beispielsweise mittels Bestrahlung mit Gammastrahlung erfolgen. Die Sterilisierung kann vorteilhaft ebenso wie die Evakuierung des Gehäuses 102 bereits vom Hersteller bei der Herstellung der Inline-Sensoranordnung 107 durchgeführt werden.

Zur thermischen Entkopplung des Sensorelements 104 von der Außenseite des Gehäuses 102 kann bereits vor oder auch nach der Integration der Inline-Sensoranordnung 107 in den Prozessbehälter 108 das von dem Gehäuse 102 eingeschlossene Volumen mittels einer mit dem Konnektor 101 verbundenen Vakuumpumpe evakuiert werden, wobei ein Druck von weniger als 100 mbar in dem Gehäuse 102 erzeugt wird. Anschließend kann der Prozessbehälter 108 zusammen mit der integrierten Inline-Sensoranordnung 107 sterilisiert werden, beispielsweise mittels Heißdampfsterilisation. Der Heißdampf wirkt dabei nur auf die Außenseite des Gehäuses 102 ein. Die Evakuierung des Gehäuses 102 bewirkt zusätzlich zu den isolierenden Eigenschaften der Gehäusewandung eine thermische Isolierung des Sensorelements 105 gegenüber der Gehäuseaußenseite. So treten bei einer Hitzesterilisation, bei der die Gehäuseaußenseite, d.h. die nach außen gerichtete Wandfläche des Gehäuses 102, einem Sterilisationsmedium von einer Temperatur von mindestens 110 °C ausgesetzt ist, am Ort des Sensorelements Temperaturen von < 80°C, bevorzugt < 40°C auf. Diese beeinträchtigen die Aktivität der biologischen Erkennungselemente des Sensorelements 102 und damit die Funktionalität des Sensors nicht oder in einem Maße, dass trotz Aktivitätsreduktion auf bis zu 10 % die Sensitivität des Sensors ausreichend ist, um den jeweiligen Prozess bzw. das durchströmende Prozessmedium zu überwachen.

Nach Beendigung der Sterilisation und nach dem Abkühlen der Gehäuseaußenseite des Gehäuses 102 bzw. der Umgebung der Inline-Sensoranordnung 107 auf weniger als 80°C, bevorzugt weniger als 60 °C oder gar als 40°C wird ein Kontakt zwischen dem Sensorelement 105 bzw. der Kammer 114 und dem Inneren des Prozessbehälters 108 hergestellt, um das Sensorelement 105 aseptisch in den Prozessbehälter 108 einzubringen und so das Erfassen von Messwerten in einem in dem Prozessbehälter 108 enthaltenen oder durch den Prozessbehälter 108 strömenden Messmedium zu ermöglichen. Im vorliegenden Beispiel ist die stirnseitige Wand 109 des Gehäuses 102 so dünn ausgestaltet, dass sie durch eine mechanische Krafteinwirkung durchstoßen werden kann. Das der Wand 109 zugewandte Ende des Sensorelementträgers 104 mit dem Sensorelement 105 weist eine Spitze oder Kante auf. Der Sensorelementträger 104 ist axial beweglich gelagert, beispielsweise mittels eines Kugelschreiber-Mechanismus, wobei der Sensorelementträger 104 relativ zu der Wand 109 so weit in axialer Richtung beweglich ist, dass die frontseitige Kante oder Spitze des Sensorelementträgers 104 die Wand 109 durchstößt und soweit über das frontseitige Ende des Gehäuses 102 hinaus in den Prozessbehälter 108 bewegt wird, dass das Sensorelement 105 in den Prozessbehälter 108 hineinragt. In dieser Stellung kann die Inline-Sensoranordnung 107 dazu dienen, die zu erfassende Messgröße eines in dem Prozessbehälter 108 enthaltenen oder diesen durchströmenden Prozessmediums zu überwachen.

Bereits bei der Herstellung der Inline-Sensoranordnung 107, bei der das Sensorelement 105 auf dem Sensorelementträger 104 in das Gehäuse 102 bzw. in die in dem Gehäuse 102 gebildete Kammer 114 dicht eingeschlossen wird, kann das Gehäuse 102 bzw. die Kammer 114 zunächst evakuiert und anschließend das Gehäuseinnere mit dem Sensorelement 105 sterilisiert werden, z.B. durch Gammastrahlung. Ein Anwender muss bei Inbetriebnahme die Inline-Sensoranordnung 107 dann nur noch in eine Wandung eines Prozessbehälters integrieren und kann gleich die Hitzesterilisation durchführen.
Um eine Zerstörung der biologischen Erkennungselemente während einer Hitzesterilisierung des Prozessbehälters 108 mit der darin integrierten Inline-Sensoranordnung 107 zu verhindern, dient nach dem hier beschriebenen Ausführungsbeispiel die thermische Entkopplung des Sensorelements 105 von der dem Sterilisationsmedium, hier Heißdampf, ausgesetzten Gehäuseaußenseite der Inline-Sensoranordnung 107. Vorteilhaft können in Abwandlungen der hier beschriebenen Inline-Sensoranordnung 107 zusätzlich zu dieser thermischen Entkopplung Maßnahmen getroffen werden, um die relative Feuchte innerhalb der Kammer 114 unterhalb eines Werts von 77 % oder weniger während der Hitzesterilisation zu halten. Wie weiter oben erläutert, kann so eine Denaturierung der Erkennungselemente ebenfalls verhindert werden. Denkbare Maßnahmen zur Vermeidung einer zu hohen relativen Feuchte in der Kammer 114 sind beispielsweise die Verwendung von Materialien mit einer geringen Wasserdampfdurchlässigkeit der die Kammer 114 umgebenden Komponenten, wie anhand des in Fig. 1 dargestellten Ausführungsbeispiels beschrieben, das Zugeben eines Trocknungsmittels in die Kammer 114 bei der Herstellung der Inline-Sensoranordnung 107 oder das Zuleiten eines wasserfreien oder wasserarmen Fluids, insbesondere von reinem Stickstoff oder Luft mit einem Wassergehalt von weniger als 50 ppmv H₂O, oder sogar weniger als 5 ppmv H₂O über einen Sterilfilter, den das Fluid vor dem Eintritt in die Kammer 114 passiert, z.B. über den Konnektor 101.

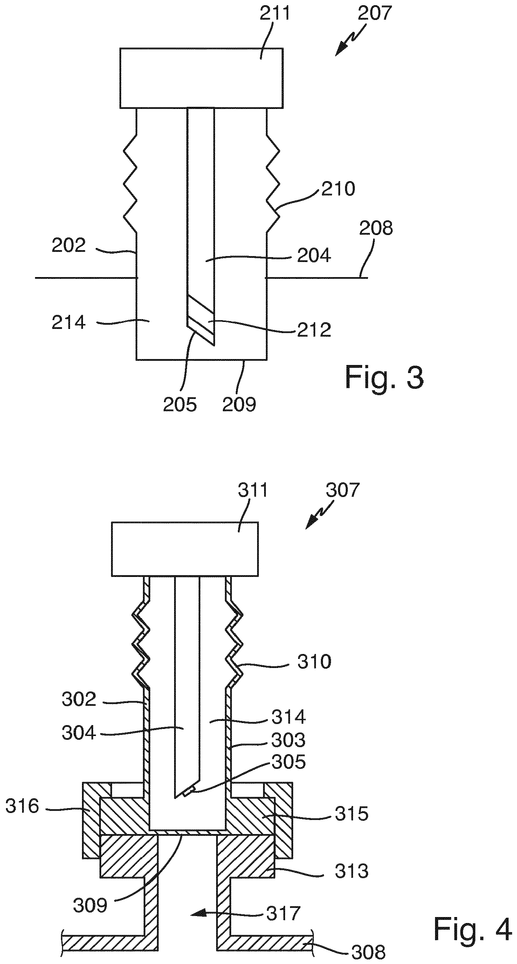

In Fig. 3 ist schematisch ein weiteres Ausführungsbeispiel einer Inline-Sensoranordnung 207 dargestellt. Diese Inline-Sensoranordnung 207 umfasst einen Sensor mit einem Sensorträger 204, auf dem ein analytsensitives Sensorelement 205 angeordnet ist. Diese können in gleicher Weise ausgestaltet sein, wie der Sensor bzw. der Sensorelementträger 4 und das Sensorelement 5 der zuvor anhand von Fig. 1 beschriebenen Inline-Sensoranordnung 7. Das Sensorelement 205 ist über elektrische Leitungen, die innerhalb eines in dem Sensorelementträger 204 gebildeten Kanals geführt sein können, mit einer Messschaltung verbunden, welche in einem Elektronikgehäuse 211 angeordnet ist. Die Messschaltung ist dazu ausgestaltet, ein von der durch das Sensorelement 205 erfassten Messgröße abhängiges Messsignal zu erzeugen und an eine übergeordnete Einheit, z.B. einen Messumformer, auszugeben. Die Messschaltung und die übergeordnete Einheit können beispielsweise über ein Kabel oder über eine Funkverbindung miteinander verbunden sein.

Der Sensorträger 204 mit dem Sensorelement 205 ist im hier gezeigten Ausführungsbeispiel starr mit dem Elektronikgehäuse 211 verbunden. Das Elektronikgehäuse 211 verschließt rückseitig ein im Wesentlichen zylindrisches Gehäuse 202, das den Sensorelementträger 204 mit dem Sensorelement 205 umgibt und in einer Kammer 214 gasdicht einschließt. Dieses Gehäuse 202 ist mittels einer nicht näher dargestellten Anschlussvorrichtung dicht in einer Wandung eines Prozessbehälters 208 befestigt, so dass die Inline-Sensoranordnung 207 in den Prozessbehälter 208 integriert ist. Das Gehäuse 202 schottet das Sensorelement 205 und den Sensorelementträger 204 durch Einschluss in der Kammer 214 vollständig gegenüber dem Prozessbehälter 208 ab.

Das Gehäuse 202 umfasst einen als Faltenbalg 210 ausgestalteten Wandungsbereich. Der Faltenbalg 210 kann in der Weise kontrahiert werden, dass die Differenz zwischen der (in axialer Richtung gemessenen) Länge des Gehäuses 202 bei entspanntem Zustand des Faltenbalgs 210 und der Länge des Gehäuses 202 bei maximal kontrahiertem Faltenbalg 210 größer ist als der Abstand zwischen der stirnseitigen, dem Prozessbehälter 208 zugewandten Wand 209 des Gehäuses 202 und dem Sensorelement 205, wobei dieser Abstand einer in axialer Richtung zwischen der Wand 209 und dem am weitesten von der Wand 209 entfernt angeordneten Punkt des Sensorelements 205 verlaufenden Strecke entspricht. Die Inline-Sensoranordnung 207 kann zusätzlich (in Fig. 2 nicht dargestellte) Arretierungselemente aufweisen, die den Faltenbalg 210 in kontrahierter Stellung fixieren.

Die Wand 209 kann als Membran oder als relativ dünner Wandabschnitt ausgebildet sein. Beispielsweise kann es sich bei der Wand um eine feuchteundurchlässige Folie handeln, die mindestens eine metallische Lage aufweist, die eine geringe Wasserdampfdurchlässigkeit aufweist. Das der Wand 209 zugewandte Ende des Sensorelementträgers 204 kann eine Kante oder Spitze aufweisen, die dazu geeignet ist, die Wand 209 zu durchstoßen und so die Kammer 214 zum Prozessbehälter 208 hin durch Herstellen einer Verbindung zwischen dem Inneren des Gehäuses 202 und dem Inneren des Prozessbehälters 208 zu öffnen.

Zur thermischen Entkopplung des Sensorelements 205 von der Gehäuseaußenseite des Gehäuses 202 dient ein Peltier-Element 212, das mit der dem Sensorelementträger 204 zugewandten Rückseite des Sensorelements 205 in flächigem Kontakt steht. Elektrische Anschlüsse des Peltier-Elements 212 können über durch den in dem Sensorelementträger 204 gebildeten Kanal verlaufende Leitungen kontaktiert werden. Das Peltier-Element 212 kann so mittels der Messschaltung betrieben werden. Zur Wärmeableitung kann das Peltier-Element 212 mit einer Wärmesenke in Kontakt stehen. Diese kann eine innerhalb des Sensorelementträgers 204 gebildete Fluid-Kühlung umfassen. Beispielsweise kann die Fluid-Kühlung einen als Kanalstruktur innerhalb des Sensorelementträgers 204 gebildeten, fluiddurchströmten Kühlkreislauf aufweisen.

In alternativen Ausgestaltungen ist es auch möglich, das Sensorelement 205 allein mittels einer Fluid-Kühlung aktiv zu kühlen. Diese kann innerhalb des Sensorträgers und/oder innerhalb des Gehäuseinnenraums des Gehäuses 202 oder innerhalb der Wandung des Gehäuses 202 gebildet sein. In einer anderen alternativen Ausgestaltung kann die mit dem Peltier-Element 212 zusammenwirkende Wärmesenke aus einem Material mit hoher Wärmekapazität und/oder großer Oberfläche, beispielsweise in Form von Kühlblechen oder rippen gebildet sein.

Vor oder nach der Integration der Inline-Sensoranordnung 207 in eine Wandung des Prozessbehälters 208 kann das Innere des Gehäuses 202 mit dem in der Kammer 214 darin befindlichen Sensorelement 205 und dem Sensorelementträger 204 mittels Bestrahlung mit Gammastrahlung sterilisiert werden.

Bei Inbetriebnahme des Prozessbehälters 208 und der Inline-Sensoranordnung 207 kann eine Heißdampf-Sterilisation mit der in die Wandung des Prozessbehälters 208 integrierten Inline-Sensoranordnung 207 in Form eines SIP-Verfahrens durchgeführt werden. Gleichzeitig erfolgt zur thermischen Entkopplung des Sensorelements 205 von der dem Heißdampf ausgesetzten Gehäuseaußenseite, d.h. der nach außen gerichteten Wandfläche des Gehäuses 202 eine aktive Kühlung des Sensorelements 205 mittels des Peltier-Elements 212. Aufgrund der Einwirkung des Heißdampfs auf die Gehäuseaußenfläche des Gehäuses 202 erwärmt sich diese auf Temperaturen bis zu 120°C. Gleichzeitig erwärmt sich das thermisch entkoppelte Sensorelement 205 höchstens bis zu 80°C, vorzugsweise weniger als 40°C, so dass die Funktionalität des Sensorelements 205 und damit des Sensors erhalten bleibt.

Nach Beendigung der Sterilisation, insbesondere nachdem die in dem Prozessbehälter 208 herrschende Temperatur auf weniger als 60°C, vorzugsweise weniger als 40°C abgesunken ist, kann die Kühlung des Sensorelements 205 beendet werden. Zum Inkontaktbringen des Sensorelements 205 mit dem Inneren des Prozessbehälters 208 bzw. mit einem in dem Prozessbehälter 208 enthaltenen Prozessmedium, kann durch Ausübung einer in axialer Richtung wirkenden Kraft auf das Elektronikgehäuse 211 der Sensorelementträger 204 mit dem darauf angeordneten Sensorelement 205 auf die stirnseitige Wand 209 des Gehäuses 202 zu bewegt werden. Dabei wird der Faltenbalg 211 kontrahiert. Mit der stirnseitigen Kante oder Spitze des Sensorelementträgers 204 kann auf diese Weise die Wand 209 durchstoßen werden, und so das Sensorelement 205 aseptisch mit dem Inneren des Prozessbehälters 208 in Kontakt gebracht werden. Wie voranstehend beschrieben, ist der Faltenbalg 210 so ausgestaltet, dass bei vollständiger Kontraktion des Faltenbalgs 210 das Sensorelement 205 über die Länge des Gehäuses 202 hinausragt, so dass das Sensorelement 205 in Kontakt mit dem Inneren des Prozessbehälters 208 steht und in Kontakt mit einem darin befindlichen Prozessmedium Messwerte der Messgröße erfassen kann.

Aufgrund der thermischen Entkopplung des Sensorelements 205 während der Heißdampfsterilisation ist eine Beeinträchtigung der Funktionalität des Sensorelements 205 wirksam verhindert, auch wenn dieses biologische Erkennungselemente in Form von denaturierbaren Enzymen oder Proteinen umfasst. Zusätzlich oder alternativ kann die Inline-Sensoranordnung 207 derart ausgestaltet sein, dass die während der Heißdampfsterilisierung die relative Feuchte innerhalb der Kammer 214 unterhalb eines Werts von 77 % bleibt. Hierzu sind die weiter oben bereits im Zusammenhang mit den in Fig. 1 und Fig. 2 beschriebenen Ausführungsbeispielen beschriebenen Maßnahmen geeignet.

In Fig. 4 ist ein weiteres Ausführungsbeispiel einer Inline-Sensoranordnung 307 dargestellt, bei der eine thermische Entkopplung eines Sensorelements 305 von einer mit dem Inneren eines Prozessbehälters 308 in Kontakt stehenden Gehäuseaußenseite der Inline-Sensoranordnung 307 dadurch erreicht wird, dass das Sensorelement von dem Prozessbehälter 308, beabstandet angeordnet ist.

Die Inline-Sensoranordnung 307 umfasst in diesem Ausführungsbeispiel ähnlich der Inline-Sensoranordnungen der zuvor beschriebenen Ausführungsbeispiele das bereits erwähnte Sensorelement 305, das beispielsweise eine mit biologischen Erkennungselementen zur spezifischen Wechselwirkung mit einem Analyten modifizierte Elektrode umfassen kann. Das Sensorelement 305 ist auf einem stabförmigen Sensorelementträger 304 angeordnet. Sensorelement 305 und Sensorelementträger 304 sind von einem Gehäuse 302 umgeben, das einen rohrförmigen Schaft 303 umfasst, welcher an einem dem Prozessbehälter 308 zugewandten Ende durch eine stirnseitige Wand 309 verschlossen ist. An seinem anderen Ende geht der rohrförmige Schaft 303 in einen Faltenbalg 310 über. Das Gehäuse 302 ist auf seiner der Wand 309 gegenüberliegenden Seite dicht verschlossen, beispielsweise mittels eines Vergusses (hier nicht eingezeichnet), so dass das Gehäuse 302 eine gasdicht abgeschlossene Kammer 314 einschließt, in der der Sensorelementträger 304 und das Sensorelement 308 eingeschlossen sind.

Die Inline-Sensoranordnung 307 weist außerdem ein Elektronikgehäuse 311 auf, in dem eine Messschaltung untergebracht ist, welche mit dem Sensorelement 305 in Verbindung steht, um elektrische Messsignale zu erzeugen und auszugeben, welche mit der durch das Sensorelement 305 erfassten Messgröße korreliert sind. Die Messschaltung kann ausgestaltet sein wie die Messschaltungen der zuvor anhand der Fig. 1 bis 3 beschriebenen Ausführungsbeispiele.

Das Gehäuse 302 weist an seinem dem Prozessbehälter 308 zugeordneten Ende einen Prozessanschluss 315 auf, der im vorliegenden Beispiel einen Flansch umfasst. Dieser Prozessanschluss 315 ist mit einem komplementären Behälteranschluss 313 des Prozessbehälters 308 verbunden, beispielsweise mittels einer Fixierung 316, z.B. einer Überwurfmutter. Der Prozessanschluss 315 und das Gehäuse 302 sind im vorliegenden Beispiel derart miteinander verbunden, dass die stirnseitige Wand 309 des Gehäuses 302 in einer Ebene mit der gegen den Behälteranschluss 313 anliegenden Fläche des Prozessanschlusses 315 liegt. Auf diese Weise kommt bei einer Hitzesterilisierung des Prozessbehälters 308 durch Einleiten eines Sterilisationsmediums in den Prozessbehälter 308 nur die stirnseitige Wand 309 mit dem Heißdampf in Kontakt, nicht aber die rohrförmige Seitenwandung 303 des Gehäuses 302 oder sonstige die Kammer 314 umgebende Komponenten der Inline-Sensoranordnung 307.

Dies wird, allgemeiner formuliert, dadurch erreicht, dass der Behälteranschluss 313, d.h. das an den Behälter angesetzte Rohr und der damit verbundene behälterseitige Flansch, einen mit dem Inneren 306 des Prozessbehälters 308 kommunizierenden Anschlussraum 317 umgibt, der an seinem vom Prozessbehälter 308 abgewandten Ende durch die Wand 309 verschlossen wird. Auf diese Weise ist sichergestellt, dass ein in den Prozessbehälter 308 gelangendes Sterilisationsmedium nur mit der Wand 309, nicht aber mit den übrigen mit der Kammer 314 in Kontakt stehenden Komponenten der Inline-Sensoranordnung 307 in Kontakt kommt. Der Prozessanschluss 315 ist also derart mit dem Gehäuse 302 der Inline-Sensoranordnung 307 verbunden, dass das Sensorelement 305 auf der von dem Prozessbehälter 308 abgewandten Seite der Gehäusewandung und somit außerhalb des Anschlussraums 317 angeordnet ist, wenn der Prozessanschluss 315 und der Behälteranschluss 313 miteinander verbunden sind. In einer solchen Anordnung wird das axial von der Wand 309 beabstandet angeordnete Sensorelement 305 weniger stark erwärmt als bei einer Anordnung wie beispielsweise in Fig. 1 dargestellt, bei der auch die Seitenwandung 3 des Gehäuses 2 bei einer Hitzesterilisierung des Prozessbehälters 8 in unmittelbarem Kontakt mit einem hierzu verwendeten Sterilisationsmedium kommt.

Die Inbetriebnahme der Inline-Sensoranordnung und das aseptische in Kontakt bringen des Sensorelements 305 mit einem in dem Prozessbehälter 308 enthaltenen Messmedium kann im Übrigen in gleicher Weise erfolgen, wie die Inbetriebnahme der anhand von Fig. 3 beschriebenen Inline-Sensoranordnung 207.

Weitere Abwandlungen und Ausgestaltungen der erfindungsgemäßen Inline-Sensoranordnung sind denkbar. Beispielsweise kann das den Sensorelementträger und das Sensorelement umgebende und während der Sterilisation des Prozessbehälters von dessen Innerem trennende Gehäuse auch so ausgestaltet sein, dass das Herstellen einer Verbindung zwischen dem Sensorelement und dem Innenraum des Prozessbehälters reversibel erfolgt. Hierzu kann die Gehäusewandung beispielsweise eine Öffnung umfassen, die mittels eines Deckels oder einer Kappe reversibel verschließbar ist, um das Sensorelement gegenüber dem Prozessbehälter abzuschotten, und die geöffnet werden kann, wenn eine Verbindung zwischen dem Sensorelement und dem Inneren des Prozessbehälters hergestellt werden soll. Das Gehäuse kann auch durch eine Behandlungskammer einer Tauch- oder Wechselarmatur oder einer Schleuseneinrichtung ausgebildet sein, die derart ausgestaltet ist, dass das Sensorelement mit dem Sensorelementträger zur Messung in den Prozessbehälter eingefahren werden kann oder aus dem Prozessbehälter heraus in eine gegenüber dem Prozessbehälter abgeschlossene Kammer eingefahren werden kann. Die Kammer und/oder der Sensorelementträger können in diesem Fall Mittel zur thermischen Entkopplung des Sensorelements gegenüber der mit dem Inneren des Prozessbehälters in Kontakt stehenden Außenseite, d.h. der nach außen gerichteten Wandungsfläche der Kammer bzw. des Gehäuses aufweisen. In all diesen Ausgestaltungen ist eine mehrmalige Verwendung des Gehäuses zum aseptischen in Kontakt bringen eines, insbesondere hitze- und/oder feuchteempfindlichen Sensorelements mit einem Messmedium, welches in einem vorab durch hitzesterilisierten Prozessbehälter enthalten ist, ohne Ausbau der ganzen Inline-Sensoranordnung möglich, d.h. dasselbe Gehäuse kann für mehrere Produktionschargen mit zwischen den einzelnen Chargen durchgeführter Sterilisation des Prozessbehälters in der Wandung des Prozessbehälters verbleiben. Es ist dabei insbesondere möglich, dass das Gehäuse permanent in dem Prozessbehälter integriert ist, während der Sensorelementträger und das darauf angeordnete Sensorelement gegen einen baugleichen, anderen Sensorelementträger und ein darauf angeordnetes Sensorelement austauschbar sind.

## Patentansprüche

1. Inline-Sensoranordnung zur Erfassung von Messwerten einer einen Analytgehalt eines Messmediums repräsentierenden Messgröße, umfassend:
- einen Sensor, welcher dazu ausgestaltet ist, ein mit der Messgröße korreliertes Messsignal zu erzeugen und auszugeben,
wobei der Sensor mindestens ein zum Kontakt mit dem Messmedium vorgesehenes steriles Sensorelement aufweist; und
- ein das mindestens eine Sensorelement umgebendes Gehäuse, das das Sensorelement in einer gegenüber einer Umgebung des Gehäuses dicht verschlossenen Kammer einschließt, und
wobei die Kammer in ihrem Innern ein Gasvolumen enthält, das derart ausgestaltet ist, dass ein Einfluss von Schadsubstanzen, insbesondere von reaktiven Stickstoff- und/oder Sauerstoff-Spezies, auf das mindestens eine Sensorelement weitgehend verhindert wird.

2. Inline-Sensoranordnung nach Anspruch 1,
wobei das Gasvolumen im Innern der Kammer weitgehend aus einem Schutzgas, vorzugsweise aus einem oder mehreren Edelgasen, gebildet wird und
wobei das Gasvolumen im Innern der Kammer Sauerstoff und/oder Stickstoff in einem Volumenanteil von jeweils weniger als 1 Volumen-%, bevorzugt jeweils weniger als 0,5 Volumen-%, weiter bevorzugt jeweils weniger als 0,1 Volumen-%, enthält.

3. Inline-Sensoranordnung nach einem der Ansprüche 1 oder 2,
wobei im Innern der Kammer Mittel zur Eliminierung von Sauerstoff und/oder der Eliminierung oder Adsorption von gasförmigen Schadsubstanzen, insbesondere zur Eliminierung/Adsorption von Stickoxiden und/oder reaktiven Stickstoff- und/oder Sauerstoffspezies, angeordnet sind.

4. Inline-Sensoranordnung nach einem der Ansprüche 1 bis 3,
wobei das Gehäuse und die darin eingeschlossene Kammer derart ausgestaltet und die Kammer gegenüber der Umgebung derart abgedichtet ist, dass bei einer Hitzesterilisation des Gehäuses von außen bei einer Temperatur von 110°C über einen Zeitraum von 15 min die innerhalb des Gehäuses herrschende relative Feuchte einen Wert von 77 %, bevorzugt von 23 %, weiter bevorzugt von 3 %, noch weiter bevorzugt von 1 % nicht übersteigt.

5. Inline-Sensoranordnung nach einem der Ansprüche 1 bis 4,
wobei das Gehäuse eine aus einer oder mehreren Gehäusekomponenten gebildete Wandung umfasst, die die Kammer dicht einschließt, wobei eine mittlere Wasserdampfdurchlässigkeit der Wandung bei einer Temperatur von 110°, einer zwischen der Kammer und der Umgebung der Wandung herrschenden Druckdifferenz von weniger als 5 bar und einer Differenz der in der Kammer und in der Umgebung der Wandung herrschenden relativen Feuchten von mehr als 67% weniger als 420 g/(m²d), bevorzugt von weniger als 125 g/(m²d), weiter bevorzugt von weniger als 15 g/(m²d), noch weiter bevorzugt von weniger als 6 g/(m²d) beträgt.

6. Inline-Sensoranordnung nach einem der Ansprüche 1 bis 5,
wobei die Kammer ein Trocknungsmittel enthält.

7. Inline-Sensoranordnung nach einem der Ansprüche 1 bis 6,
wobei das mindestens eine Sensorelement mindestens zeitweise von der Umgebung der Gehäuseaußenseite des Gehäuses thermisch derart entkoppelt oder entkoppelbar ist, dass während der Einwirkung eines eine Temperatur von 110°C aufweisenden Mediums auf mindestens einen Teilbereich der Gehäuseaußenseite über einen Zeitraum von 15 min die Temperatur des Sensorelements ausgehend von einer Anfangstemperatur des Sensorelements von 25°C bei Beginn dieses Zeitraums um weniger als 55°C, vorzugsweise um weniger als 35°C, weiter bevorzugt um weniger als 10°C, ansteigt.

8. Inline-Sensoranordnung nach einem der Ansprüche 1 bis 7,
wobei die Inline-Sensoranordnung mit mindestens einem Temperaturfühler/-sensor und/oder einen Feuchtesensor versehen ist, die dazu ausgestaltet sind, die innerhalb der Kammer herrschende Temperatur und/oder die relative Feuchte repräsentierende Messwerte zu erfassen.

9. Inline-Sensoranordnung nach einem der Ansprüche 1 bis 8,
wobei das Gehäuse einen Wandungsbereich aufweist, welcher dazu ausgestaltet ist, das Sensorelement in Kontakt mit der Umgebung des Gehäuses zu bringen und/oder wobei das Sensorelement und der Wandungsbereich relativ zueinander in der Weise beweglich sind, dass das Sensorelement aus dem Gehäuse heraus verschoben werden kann.

10. Inline-Sensoranordnung nach einem der Ansprüche 1 bis 9,
wobei das Sensorelement ein biologische Erkennungselemente, insbesondere mindestens ein unter Beibehaltung von mindestens 10% seiner Funktionalität lyophilisierbares Enzym, aufweisendes, insbesondere als ein enzym-basierter Glukosesensor ausgestaltetes, Sensorelement ist.

11. Verfahren zur Herstellung einer Inline-Sensoranordnung nach einem der Ansprüche 1 bis 10, umfassend:
- Herstellen einer Inline-Sensoranordnung mit einem Sensor, welcher dazu ausgestaltet ist ein mit der Messgröße korreliertes Messsignal zu erzeugen und auszugeben, wobei der Sensor mindestens ein zum Kontakt mit dem Messmedium vorgesehenes Sensorelement aufweist, und mit einem das Sensorelement und mindestens einen Abschnitt des Sensorelements umgebenden Gehäuse, welches das Sensorelement in einer gegenüber einer Umgebung des Gehäuses dicht verschlossenen Kammer einschließt; und
- Sterilisieren des in der Kammer angeordneten mindestens einen Sensorelements der Inline-Sensoranordnung mittels Bestrahlung durch Beta- oder Gammastrahlung.

12. Verfahren nach Anspruch 11,
wobei das Herstellen der Inline-Sensoranordnung umfasst:
- Gasdichtes Verschließen der Kammer, wobei eine in der Kammer nach dem Verschließen vorliegende Luftfeuchtigkeit so bemessen ist, dass bei einer Hitzesterilisation des Gehäuses von außen bei einer Temperatur von 110°C über einen Zeitraum von 15 min die innerhalb der Kammer herrschende relative Feuchte einen Wert von 77 %, bevorzugt von 23 %, weiter bevorzugt von 3 %, noch weiter bevorzugt von 1 % nicht übersteigt.

13. Verfahren zur Inbetriebnahme einer Inline-Sensoranordnung nach einem der Ansprüche 1 bis 12, wobei das Verfahren die Schritte umfasst:
- Durchführen einer Hitzesterilisierung mindestens eines eine Gehäuseaußenseite des Gehäuses umfassenden Teils der Inline-Sensoranordnung;
- Öffnen des Gehäuses nach Beendigung der Hitzesterilisierung; und
- In Kontakt bringen des Sensorelements mit dem Messmedium.

14. Verfahren nach Anspruch 13,
wobei die Inline-Sensoranordnung vor dem Durchführen der Hitzesterilisierung dicht in eine Wandung eines Prozessbehälters integriert wird, und die Hitzesterilisierung der Inline-Sensoranordnung in einem einzigen Verfahrensschritt zusammen mit einer Hitzesterilisierung des Prozessbehälters durchgeführt wird, und wobei das Gehäuse nach Beendigung der Hitzesterilisierung zum Prozessbehälter geöffnet wird.

15. Verfahren nach einem der Ansprüche 13 bis 14,
wobei die Inline-Sensoranordnung mindestens einen Temperaturfühler/-sensor aufweist und wobei der Temperaturverlauf des mindestens einen Sensorelements während der Hitzesterilisierung durch den mindestens einen Temperaturfühler/-sensor der Inline-Sensoranordnung überwacht wird.
